# EUROPEAN PATENT SPECIFICATION

(11) **EP 1 755 456 B1**
(45) Date of publication and mention of the grant of the patent: **21.10.2009**
(21) Application number: 05746249.1
(22) Date of filing: 06.05.2005
(51) Int. Cl.: A61B 5/15

(54) **CONTACT ACTIVATED LANCET DEVICE**
DURCH KONTAKT AKTIVIERTE LANZETTENVORRICHTUNG
DISPOSITIF LANCETTE ACTIONNE PAR CONTACT

(30) Priority: 07.05.2004 US 569424 P; 30.11.2004 US 631846 P; 30.11.2004 US 631795 P; 07.04.2005 US 669276 P
(43) Date of publication of application: 28.02.2007
(73) Proprietor: Becton, Dickinson and Company, Franklin Lakes, New Jersey 07417 (US)
(72) Inventor: KARBOWNICZEK, Jacek, Grzegorz, PL-00-830 Warsaw (PL); RUTYNOWSKI, Wlodzinierz, PL-02-942 Warsaw (PL); WILKINSON, Bradley, North Haledon, NJ 07508 (US)
(74) Representative: Weber, Thomas
(86) International application number: PCT/US2005/015771
(87) International publication number: WO 2005/110227

(56) References cited:
- EP-A- 0 569 124
- WO-A-03/049613
- US-A- 3 741 197
- US-A- 4 577 630
- US-A- 4 637 403
- US-A- 4 653 513
- US-A1- 2002 128 608

## Description

### CROSS REFERENCE TO RELATED APPLICATIONS

The present application claims priority to U.S. Application No. 60/569,424 filed May 7, 2004, U.S. Application No. 60/631,846 filed November 30, 2004, U.S. Application No. 60/631,795 filed November 30, 2004, and U.S. Application No. 60/669,276 filed April 7, 2005.

### BACKGROUND OF THE INVENTION

### Field of the Invention

The present invention relates generally to medical puncturing devices, commonly referred to as lancets, which are used to take blood samples from patients and, more specifically, to a lancet device that is designed for ease of use with activation achieved during contact of the device in normal use.

### Description of Related Art

Lancet devices are used in the medical field for puncturing the skin of a patient to obtain a capillary blood sample from the patient. Certain diseases, such as diabetes, require that the patient's blood be tested on a regular basis to monitor, for example, the patient's blood sugar levels. Additionally, test kits, such as cholesterol test kits, often require a blood sample for analysis. The blood collection procedure usually involves pricking a finger or other suitable body part in order to obtain the blood sample. Typically, the amount of blood needed for such tests is relatively small and a small puncture wound or incision normally provides a sufficient amount of blood for these tests.

Various lancet devices are commercially available to hospitals, clinics, doctors' offices, and the like, as well as to individual consumers. Such devices typically include a sharp-pointed member such as a needle, or a sharp-edged member such as a blade, that is used to make a quick puncture wound or incision in the patient's skin in order to provide a small outflow of blood. It is often physiologically and psychologically difficult for many people to prick their own finger with a hand-held needle or blade. As a result, lancet devices have evolved into automatic devices that puncture or cut the skin of the patient upon the actuation of a triggering mechanism. In some devices, the needle or blade is kept in a standby position until it is triggered by the user, who may be a medical professional in charge of drawing blood from the patient, or the patient himself or herself. Upon triggering, the needle or blade punctures or cuts the skin of the patient, for example on the finger. Often, a spring is incorporated into the device to provide the "automatic" force necessary to puncture or cut the skin of the patient.

It is of the utmost importance in the medical field that such medical puncturing devices or lancets are in a sterile condition before use. Today, generally without exception, medical puncturing devices or lancets are manufactured and packaged in a sterilized condition before they are distributed to medical professionals and members of the public who have a need for such devices. The sterile packaging maintains the sterility of the device, ensuring that the surrounding environment does not contaminate it until use. In addition, it is also of increasing importance that the user or another person does not come into contact with the needle or blade after use of the device. With the concern over blood-borne diseases, medical professionals are required to take great care with medical devices that come into contact with the blood of patients. Thus, an important aspect of lancet design involves preventing the needle or blade of the device from wounding the user or another person after the blood sample is drawn from the patient. Once used, the needle or blade should be shielded to prevent the needle or blade from wounding the user or another person handling the device. Moreover, the lancet device should be disposable to eliminate the chances of disease transmission due to the needle or blade being used on more than one person. In this regard, the lancet device should ideally be designed for one firing, and have safety features to prevent reuse.

Advances have been made in recent years to increase safety in operating and handling used lancet devices. For example, lancet devices are currently available which are single shot devices that feature automatic ejection and retraction of the puncturing or cutting element from and into the device. Examples of such medical puncturing devices are disclosed in U.S. Patent Nos. 6,432,120; 6,248,120; 5,755,733; and 5,540,709.

U.S. Patent No. 6,432,120 to Teo discloses a lancet device that includes a lancet holder which contains a spring-loaded lancet structure. The spring-loaded lancet structure includes a single spring that effects the ejection and retraction of a lancet needle upon the triggering of the structure. U.S. Patent No. 6,248,120 to Wyszogrodzki discloses a lancet device comprised of a housing, a shielding portion, a piston with a puncturing tip, and drive and return springs that eject and retract the piston, respectively, upon the breakage of internal wing elements in the housing. U.S. Patent No. 5,755,733 to Morita discloses a lancet device that includes a combined holder and lancet structure. The lancet structure includes a lancet member with a puncturing tip and a compressible spring member that causes the lancet member to puncture the skin of a patient upon actuation of a pair of actuating arms.

U.S. Patent No. 5,540,709 to Ramel discloses a lancet device that includes a housing enclosing a slidable trigger, which is used to trigger a compressed spring that powers a piercing lancet member to pierce the skin of a patient. The housing includes a pair of internal fingers that engage and hold the body of the lancet member, which are then released of engagement with the lancet member body by axial force applied by the user to the slidable trigger. Other medical puncturing devices or lancets known in the art are disclosed in U.S. Patent Nos. 4,869,249 and 4,817,603. The devices disclosed in these references include a cap that is used to protect a needle or to keep the needle sterile.
U.S. 4,577,630 discloses a lancet device essentially corresponding to the first part of claim 1. This lancet device has a sled which releasably engages a lancet so that the lancet point may stand outwardly from a distal end of the sled. The sled is contained in a housing. A spring is provided for biasing the distal end of the sled towards the distal end of the housing. A cocking mechanism releasably holds the sled in a retracted position whereby the spring remains in a biased condition. A trigger mechanism for releasing the cocking mechanism is provided. This trigger mechanism comprises a contact surface for contacting a patient and a sear member acting against a sear surface of the sled. A cam is provided for guiding the trigger sear member for disengaging from the sled so that the sled with the lancet is propelled by the compression spring toward the distal end of the housing.

### SUMMARY OF THE INVENTION

In view of the foregoing, a need generally exists in the medical field for a medical puncturing device that is easy for a user to manipulate and use while ensuring sterility before use and safe and secure disposal after use. Additionally, a need exists in the medical field for a simple, reliable, self-activating, and disposable medical puncturing device for use in collecting blood samples.
The lancet device of the present invention is defined by claim 1. It is **characterized in that** the lever element comprises a class 1 type lever, wherein movement of the housing toward the lever element causes a portion of the lever element to pivot about the fulcrum thereby moving the lancet structure toward the rearward end of the housing to at least partially compress the drive spring and releasing the interference engagement between the lever element and the lancet structure, permitting the drive spring to drive the lancet structure through the housing toward the puncturing position.

A lancet device in an embodiment of the invention generally includes a housing and a lancet structure having a puncturing element, with the lancet structure disposed within the housing and adapted for movement between a retaining or pre-actuated position wherein the puncturing element is retained within the housing, and a puncturing position wherein the puncturing element extends through a forward end of the housing. The lancet device further includes a drive spring for biasing the lancet structure toward the puncturing position. The drive spring may be disposed between a rearward end of the housing and the lancet structure, and may be a separate structure or may be integrally formed with one or both of the housing and/or the lancet structure. The lancet device further includes a lever element pivotal about a fulcrum providing interference engagement with the lancet structure and retaining the lancet structure in the retracted position against the bias of the drive spring. The lever element may include a retaining hub including a pivotal lever in interference engagement with the lancet structure and pivotal about a fulcrum of the retaining hub. Movement of the housing and the retaining hub with respect to each other, such as axial or longitudinal movement, causes the lever to pivot about a fulcrum, thereby releasing the lever from interference engagement with the lancet structure and, typically, moving the lancet structure toward the rearward end of the housing to at least partially compress the drive spring. With the lancet structure released from the lever, the drive spring drives the lancet structure through the housing toward the puncturing position.

The lever may include a shoulder in interference engagement with the lancet structure and a contact surface for engagement with an internal contact within the housing. The housing may include an internal contact therein for pivoting of the lever, which may be an integrally formed cam surface for cooperating engagement with the contact surface of the lever. The lever is a class 1 type lever, with a pivoting point or fulcrum between the load element and the force element, such as a see-saw type of lever. Desirably, the lever is a wedge pivotally hinged to the retaining hub forming a pivot hinge defining the fulcrum for cooperative pivoting of the shoulder and the contact surface about the hinge or fulcrum. Moreover, the retaining hub may include an annular rim, with the lever pivotally hinged to the annular rim.

In a further embodiment of the invention, a lancet device includes a housing and a lancet structure adapted for axial or longitudinal movement through the housing between a pre-actuated position with a puncturing element of the lancet structure retained within the housing and a puncturing position with the puncturing element extending through a forward end of the housing. A drive spring biases the lancet structure toward the puncturing position, and a retaining hub retains the lancet structure in the pre-actuated position against the bias of the drive spring. The retaining hub includes a lever pivotal about a fulcrum, with the lever in interference engagement with the lancet structure. An actuator is adapted to pivot the lever about the fulcrum to release the lever from interference engagement with the lancet structure, thereby permitting the drive spring to drive the lancet structure to the puncturing position.

The actuator may include an actuator element extending through and into the housing, such as a push button element extending through a rearward end of the housing which is axially movable with respect to the housing to cause the actuator element to pivot the lever about the fulcrum. Alternatively, the actuator may include an internal contact within the housing such as an internal contact integrally formed within the housing, such that axial movement of the housing toward the retaining hub causes the internal contact within the housing to pivot the lever about the fulcrum.

Desirably, the lancet device further includes a shield extending through the forward end of the housing and axially or longitudinally movable with respect to the housing, with the retaining hub adjacent the rearward end of the shield. The lancet structure may be retained by the retaining hub at the rearward end of the shield. In this manner, relative axial or longitudinal movement of the rearward end of the shield and the rearward end of the housing toward each other causes the internal contact within the housing to pivot the lever of the retaining hub, thereby actuating the device by releasing the interference engagement between the lever and the lancet structure. Desirably, the lancet structure and the shield include corresponding guiding surfaces for guiding the lancet structure axially or longitudinally through the shield.

In a further embodiment, a lancet device includes a housing having a rearward end and a forward end having an opening extending therethrough, and a shield movable through the opening of the forward end of the housing, such as axially or longitudinally, with the shield including a lever element , such as a retaining hub adjacent a rearward end thereof, including a pivotal lever. A lancet structure with a puncturing element is disposed within the housing. The lancet structure is in interference engagement with the lever of the retaining hub at the rearward end of the shield, and is adapted for axial or longitudinal movement between a retaining or pre-actuated position with the puncturing element disposed within the housing, and a puncturing position with the puncturing element extending through a forward end of the shield. A drive spring is disposed between the housing and the lancet structure for biasing the lancet structure against the lever of the retaining hub, with the interference engagement between the lever and the lancet structure maintaining the lancet structure in the retaining or pre-actuated position. Axial or longitudinal movement of the shield toward the rearward end of the housing causes an internal contact within the housing to engage the lever of the retaining hub, thereby causing pivotal movement of the lever to release the interference engagement between the lever and the lancet structure. The drive spring may thereafter drive the lancet structure axially or longitudinally through the shield toward the puncturing position.

The retaining hub desirably includes an annular rim, which may be separate from and retained within the rearward end of the shield, with at least one lever pivotally supported on the annular rim. More desirably, a pair of levers are pivotally supported on opposing sides of the annular rim. The lever may further include a shoulder for interference engagement with the lancet structure, as well as a contact surface for engagement with the internal contact of the housing to cause the lever to pivot, thereby releasing the lancet structure from interference engagement with the shoulder. For example, the internal contact of the housing may include an integrally formed cam surface for cooperating engagement with the contact surface of the lever. The lever may be in the form of a wedge pivotally hinged to the retaining hub forming a pivot hinge for cooperative pivoting of the shoulder and the contact surface.

The lancet device may further include a retraction spring for retracting the lancet structure within the shield after the drive spring drives the lancet structure axially through the shield toward the puncturing position. For example, the retraction spring may be a compression spring positioned within the forward end of the shield for compression between the lancet structure and the forward end of the shield. The biasing force of the compression spring between the forward end of the shield and the lancet structure should exceed the biasing force of the drive spring between the rearward end of the housing and the lancet structure after the drive spring drives the lancet device to the puncturing position. In this manner, the retraction spring may retract the lancet structure within the shield and/or the housing, thereby maintaining the puncturing element therein. The shield and housing may further include locking structure extending therebetween for maintaining the shield in fixed relation to the housing after the drive spring drives the lancet structure through the shield toward the puncturing position.

In yet a further embodiment, an improved lancet device includes a housing and a lancet axially or longitudinally movable within the housing and retained within the housing against a bias of a drive spring which biases the lancet toward a puncturing position in which a puncturing element extends through a forward end of the housing. The improved lancet device includes a retaining hub, such as an annular structure, having at least one lever in interference engagement with the lancet for retaining the lancet within the housing against the bias of the drive spring, with the lever being pivotal about a fulcrum. Pivoting of the lever about the fulcrum releases the lancet from interference engagement with the lever to permit the drive spring to drive the lancet axially or longitudinally toward the puncturing position. The lever and fulcrum may be provided on one side of a plane dissecting the retaining hub at a cross section to an axis defined by the lancet.

In one variation of this embodiment, axial or longitudinal movement of the housing and the retaining hub with respect to each other causes an internal contact within the housing to pivot the lever about the fulcrum. This may be accomplished through a shield extending through a forward end of the housing and axially or longitudinally moveable with respect to the housing, with the retaining hub adjacent a rearward end of the shield. At least a portion of the lancet is axially or longitudinally moveable through the shield and the retaining hub upon release of the lancet from interference engagement with the lever. In a further variation, an actuator having an actuation element extends through and into the housing, desirably through the rearward end of the housing. Movement of the actuation element with respect to the housing causes the actuation element to pivot the lever about the fulcrum. In yet a further variation, the retaining hub may be unitary with the shield, and may be an annular structure.

A further embodiment provides an improved method of retaining a lancet structure in a pre-actuated position within a housing against the bias of a drive spring by providing a retaining hub in the form of an annular rim including a lever in interference engagement with the lancet structure. The lever is pivotal about a fulcrum, such that pivoting of the lever about the fulcrum releases the interference engagement between the lever and the lancet structure, permitting the drive spring to drive the lancet structure toward a puncturing position with a puncturing element extending through a forward end of the housing.

Yet a further embodiment provides a method of actuating a lancet device. The method includes providing a lancet device comprising a housing, a lancet structure disposed within the housing and including a puncturing element retained within the housing, a drive spring biased against the lancet structure, such as between a rearward end of the housing and the lancet structure, and a retaining hub retaining the lancet structure within the housing against the bias of the drive spring through a pivotal lever in interference engagement with the lancet structure. To actuate the device, the lever is contacted with an actuator to pivot the lever, thereby sequentially causing the lancet structure to move toward the rearward end of the housing to compress the drive spring and releasing the interference engagement between the lever and the lancet structure. This release causes the drive spring to drive the lancet structure axially toward a puncturing position wherein the puncturing element extends through a forward end of the housing. Other embodiments where the drive spring is constrained from further compression are also contemplated. For example, the drive spring may reach its solid height and not permit further compression while still allowing movement of the retaining hub with respect to the housing as well as release of interference engagement between the lever and the lancet structure.

The actuator desirably includes an internal contact within the housing, with the contacting step involving axially or longitudinally displacing the housing and the retaining hub toward each other to cause the internal contact of the housing to pivot the lever. Moreover, the lancet device may further include a shield extending through the forward end of the housing and axially movable with respect to the housing, with the retaining hub adjacent the rearward end of the shield for retaining the lancet structure within the housing against the bias of the drive spring. In such an embodiment, the method further includes a displacement step involving axially or longitudinally displacing the rearward end of the shield and the rearward end of the housing relative to each other to cause the contacting step. For example, the displacement step may involve applying external pressure between a forward end of the shield and the rearward end of the housing. Also, the lever may include a shoulder in interference engagement with the lancet structure and a contact surface for engagement with the internal contact of the housing. In this manner, the contacting step involves engaging the internal contact of the housing with the contact surface of the lever to pivot the lever.

Further, the retaining hub may include an annular rim with the lever pivotally hinged to the annular rim such that the shoulder extends radially inward of the annular rim and the contact surface is at an external perimeter of the annular rim. In this manner, the contacting step involves engaging the internal contact of the housing with the contact surface of the lever at an external perimeter of the annular rim, thereby pivoting the lever by tipping the contact surface and the shoulder to release the lancet structure through the annular rim and through the shield.

The method may involve a further step of retracting the puncturing element within the housing after the puncturing element reaches the puncturing position. In particular, the lancet device may further include a compression spring positioned within the forward end of the shield. The retraction step involves compressing the compression spring between the lancet structure and the forward end of the shield through the bias of the drive spring and thereafter relaxing the compression spring. In an embodiment of the invention, the biasing force of the compression spring between the forward end of the shield and the lancet structure in a relaxed state exceeds the biasing force of the drive spring between the rearward end of the housing and the lancet structure after the drive spring drives the lancet device to the puncturing position. Accordingly, the puncturing element is retracted within the shield, thereby maintaining the lancet structure within the housing.

Further details and advantages will become apparent from the following detailed description when read in conjunction with the accompanying drawings.

### BRIEF DESCRIPTION OF THE DRAWINGS

FIG. 1 is perspective view of a lancet device in accordance with an embodiment of the present invention.

FIG. 2 is perspective view of the lancet device of FIG. 1 showing the protective cover separated from the body of the lancet device.

FIG. 3 is an exploded perspective view of the lancet device of FIG. 1.

FIG. 4A is a bottom perspective view of the main body of the housing in an embodiment of the present invention.

FIGS. 4B and 4C are top perspective views of the main body of the housing of FIG. 4A.

FIGS. 4D and 4E are opposing sectional views of the main body as shown in FIG. 4C.

FIG. 5A is a bottom perspective view of the rear cap of the housing in an embodiment of the present invention.

FIGS. 5B and 5C are opposing sectional views of the rear cap as shown in FIG. 5A.

FIG. 5D is a top perspective view of the rear cap of the housing of FIG. 5A.

FIG. 5E is a sectional view of the rear cap as shown in FIG. 5D.

FIG. 6A is a bottom perspective view of the shield in an embodiment of the present invention.

FIGS. 6B and 6C are opposing sectional views of the shield as shown in FIG. 6A.

FIG. 6D is a top perspective view of the shield of FIG. 6A.

FIGS. 6E and 6F are opposing sectional views of the shield as shown in FIG. 6D.

FIG. 7A is a top perspective view of the lancet structure in an embodiment of the present invention including an integrally molded cover post portion.

FIGS. 7B and 7C are opposing sectional views of the lancet structure as shown in FIG. 7A.

FIG. 7D is a bottom perspective view of the lancet structure of FIG. 7A.

FIG. 7E is a sectional view of the lancet structure as shown in FIG. 7D.

FIG. 8A is a top perspective view of the retaining hub in an embodiment of the present invention.

FIG. 8B is a bottom perspective view of the retaining hub of FIG. 8A.

FIG. 8C is a sectional view of the lancet structure as shown in FIG. 8B.

FIG. 8D is a cross-sectional view of the lancet structure as shown in FIG. 8B.

FIG. 8E is a top perspective view of a retaining hub in an alternate embodiment.

FIG. 9A is a bottom perspective view of the tab member in an embodiment of the present invention.

FIG. 9B is a top perspective view of the tab member of FIG. 9A.

FIGS. 9C-9F are opposing sectional views of the tab member as shown in FIGS. 9A-9B.

FIG. 10 is an exploded perspective view of the shield and the retaining hub with the lancet structure.

FIGS. 11A and 11B are front and side perspective views of the lancet device of FIG. 1.

FIG. 11C is a cross-sectional view of the lancet device taken along line B-B of FIG. 11A.

FIG. 11D is a cross-sectional view of the lancet device taken along line A-A of FIG. 11B.

FIG. 12 is a cross-sectional view of the lancet device of FIG. 11D with the tab member removed and ready for use.

FIG. 13 is a cross-sectional view of the lancet device of FIG. 11D in use, with the lever partially engaged.

FIGS. 14-15 are partial enlarged perspective views of the lancet device of FIG. 11D during use, with the rear cap removed.

FIG. 16 is a partial enlarged cut-away view of the lancet device in use in the position shown in FIG. 15.

FIG. 17 is a cross-sectional view of the lancet device of FIG. 11D in use with the lancet structure in the puncturing position.

FIG. 18 is a cross-sectional view of the lancet device of FIG. 11D after use with the lancet structure in the final retracted position.

FIG. 19 is a cross-sectional view of a lancet device in an alternate embodiment of the present invention.

FIG. 20 is a perspective view of a further embodiment of a lancet device of the present invention.

FIGS. 21A-21C are bottom, side, and end views, respectively, of a retaining hub used in the lancet device shown in FIG. 53.

FIG. 22 is a perspective view of the retaining hub shown in FIGS. 21A-21C.

FIGS. 23A-23D are longitudinal cross-sectional views of another embodiment of a medical puncturing device, showing the device prior to actuation and the operational steps for actuating the device.

### DESCRIPTION OF THE PREFERRED EMBODIMENTS

For purposes of the description hereinafter, the words "upper", "lower", "right", "left", "vertical", "horizontal", "top", "bottom", "lateral", "longitudinal", "axial", and like terms, if used, shall relate to the invention, as it is oriented in the drawing figures. Additionally, the term "distal" shall refer to the portion of the device closest the puncture end and the term "proximal" shall refer to the portion of the device opposite the distal portion. It is to be understood that the invention may assume many alternative variations and embodiments except where expressly specified to the contrary. It is also to be understood that the specific devices and embodiments illustrated in the accompanying drawings and described herein are simply exemplary embodiments of the invention.

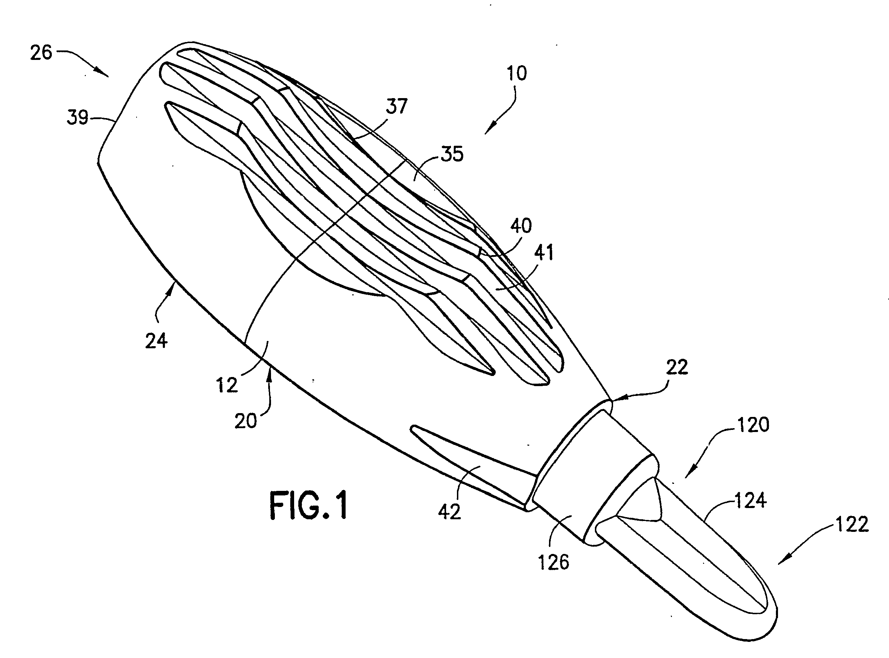

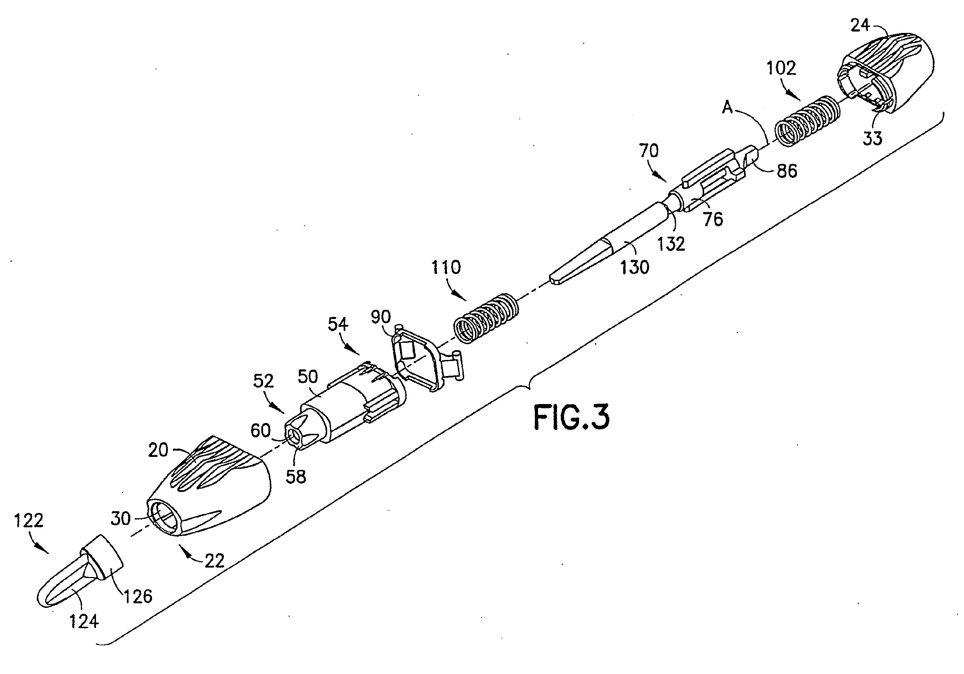

Referring to FIGS. 1-3, a lancet device **10** according to an embodiment of the invention is generally shown. The lancet device **10** generally includes a housing **12,** a shield **14** movably associated with the housing **12,** and a lancet structure **70** disposed therein. As will be discussed in greater detail herein, the shield **14** is coaxially and movably associated with the housing **12,** and is partially disposed within the housing **12,** extending partially outward from the housing **12,** with the lancet structure **70** contained within and axially or longitudinally movable through the shield **14.**

The housing **12** defines an elongated body, and is desirably formed with a main body **20** defining a distal or forward end portion **22,** and a rear cap **24** defining a proximal or rearward end portion **26.** The interior portion of housing **12** is generally open defining internal cavity **28,** which internal cavity **28** is closed at the rearward end through rear cap **24** and includes an opening **30** through the forward end portion **22,** through which the shield **14** extends, as will be discussed in further detail herein. Main body **20** and rear cap **24** may be integrally formed. Alternatively, main body **20** and rear cap **24** are separate elements which are affixed to each other to form housing **12,** which aids in assembly of lancet device **10.**

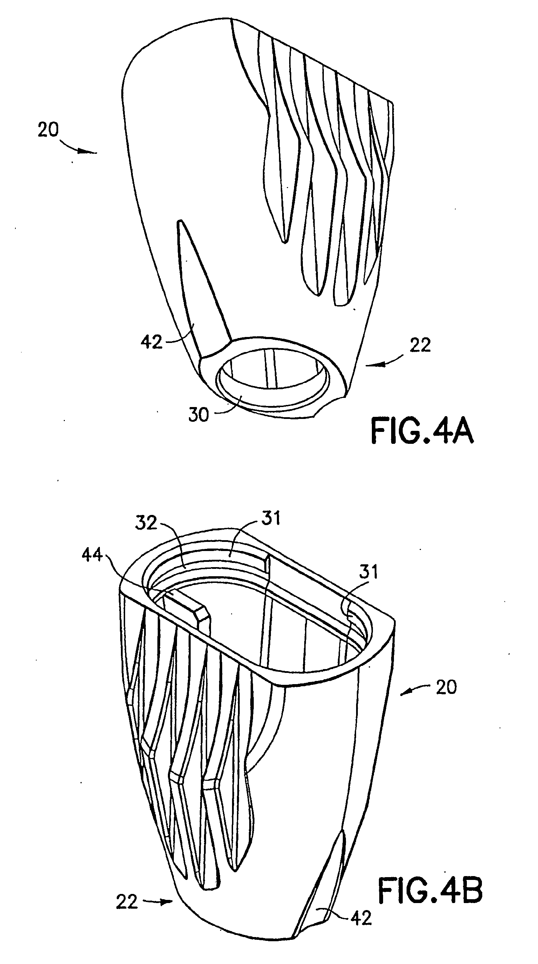

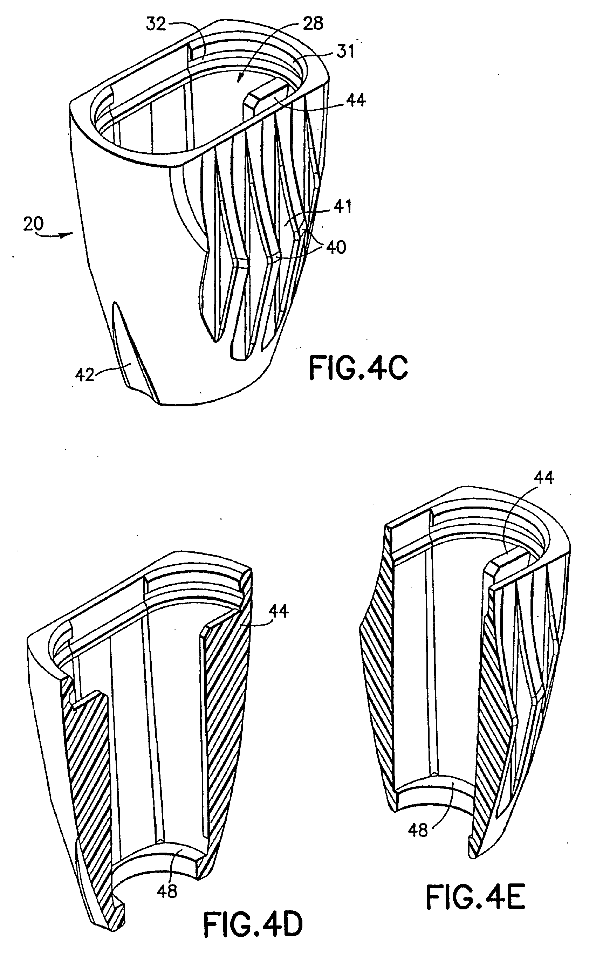

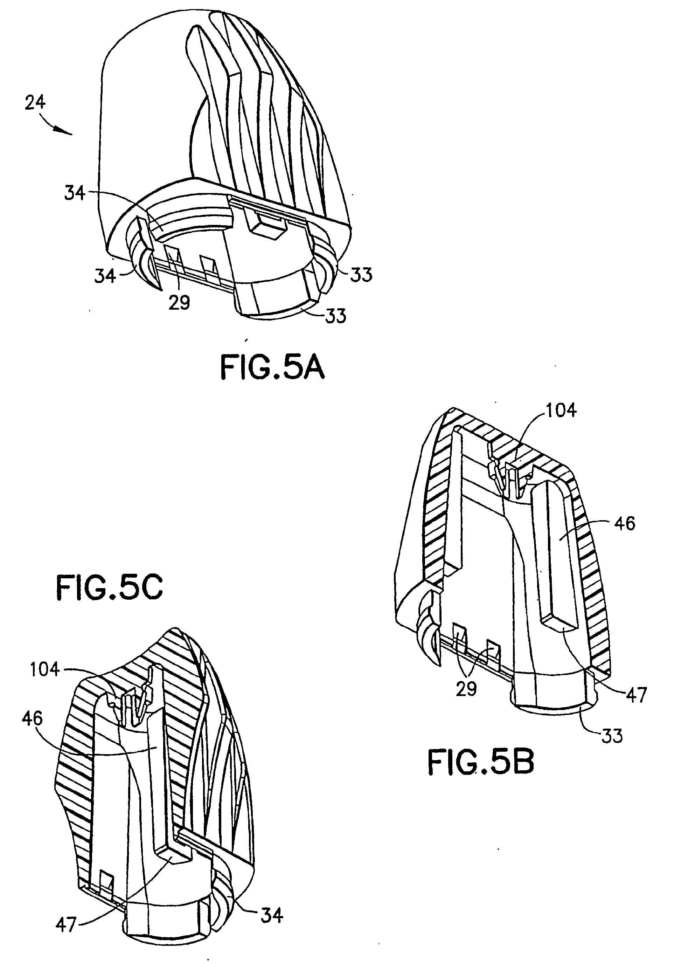

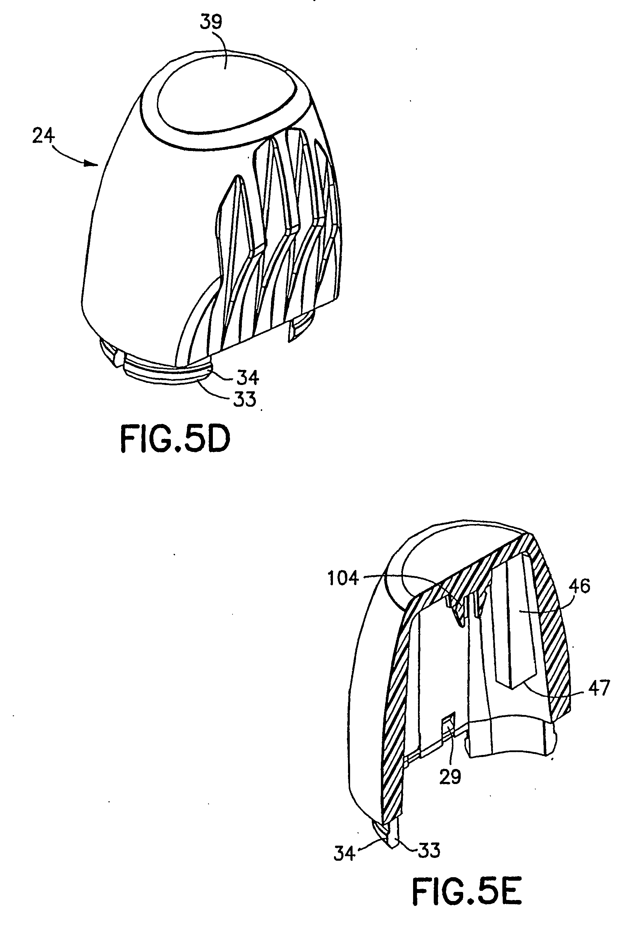

FIGS. 4A-4E and 5A-5E depict the main body **20** and rear cap **24,** respectively, in an example of such an embodiment. Main body **20** and rear cap **24** may be affixed together through an appropriate adhesive, or may include inter-engaging structure providing a mechanical attachment therebetween, such as a frictional fit or a snap fit construction. For example, main body **20** may include an annular rim **31** defining an annular groove **32,** and rear cap **24** may include an annular protrusion **33** having an annular lip **34** at mating surfaces thereof. When main body **20** and rear cap **24** are mated, annular protrusion **33** extends within the rear open end of main body **20,** with annular lip **34** snap fitting over the annular rim **31** and into the annular groove **32** of main body **20.** It should be recognized that the arrangement of such elements is merely exemplary and may be reversed, and it is contemplated that other inter-fitting engaging structure may be used to fit the main body **20** with the rear cap **24.** In an alternate embodiment, main body **20** and rear cap **24** may be an integrally formed structure, and may therefore be molded together as one component.

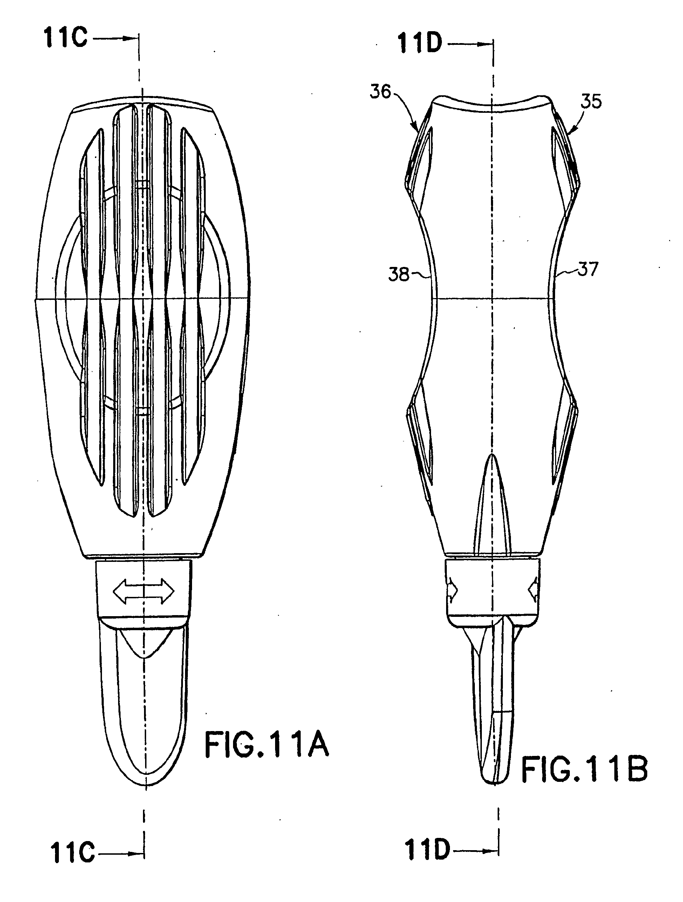

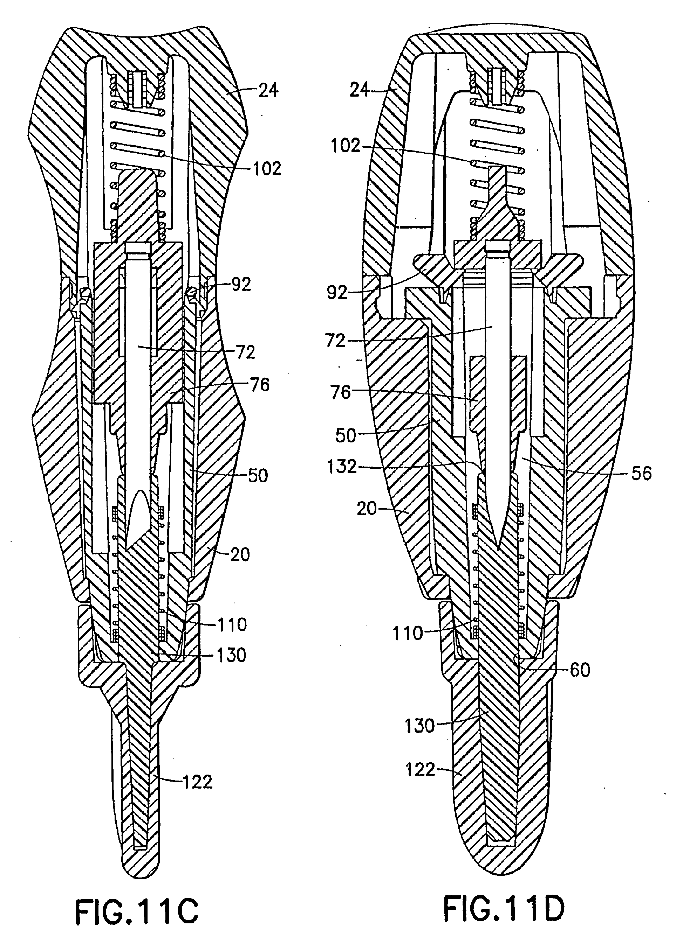

As shown in FIG. 11B, the generally elongated housing **12,** defined by main body **20** and rear cap **24,** has opposed sides **35, 36,** which may each include a surface for accommodating a user's fingers, such as finger grip indentations **37, 38.** While two opposed finger grip indentations **37, 38** are provided on the housing **12,** it will be appreciated that only one finger grip indentation **37** formed in the housing body **20** may be provided in accordance with the present invention. The finger grip indentations **37** may be formed as concave depressions or recesses on the outer surface of the housing **12.** Additionally, the rearward end **26** of housing **12,** such as the top surface of rear cap **24,** may also include a surface for accommodating a user's finger, such as rear finger grip indentation **39,** which may also be formed as a concave depression or recess. The side finger grip indentations **37, 38** and the rear finger grip indentation **39** provide ergonomically shaped surfaces that substantially conform to a user's fingertips to aid the user in manipulating the lancet device **10** and using the lancet device **10 in** a blood letting, drawing, or collection procedure, and may provide multiple finger grip positions for the user. The side finger grip indentations **37, 38** may be represented as contours formed by a hyperbola as shown in FIG. 11B. The hyperbola, for example, may include two asymptotes that intersect at a location substantially coplanar with a plane of symmetry defining the main housing. Additionally, rear cap **24** may include a contour formed by a hyperbola, as shown in FIG. 11B. The housing **12** may further include structure to generally improve the grip between the housing **12** and the user's fingertips, such as a plurality of longitudinal ribs **40** and troughs **41** extending along the housing **12** and integrally formed with the housing **12,** which may provide a visual and tactile cue to the user to instruct the user where to place his or her fingertips. The housing **12** may further include at least one and optionally two or more peripheral indentations **42** disposed at the forward end **22.** In one particular embodiment, the lancet device may be constructed in accordance with the features disclosed in Application No. 60/631,795 filed November 30, 2004, and entitled "Lancet Device" naming Bradley Wilkinson as inventor, the entirety of which is incorporated herein by reference thereto.

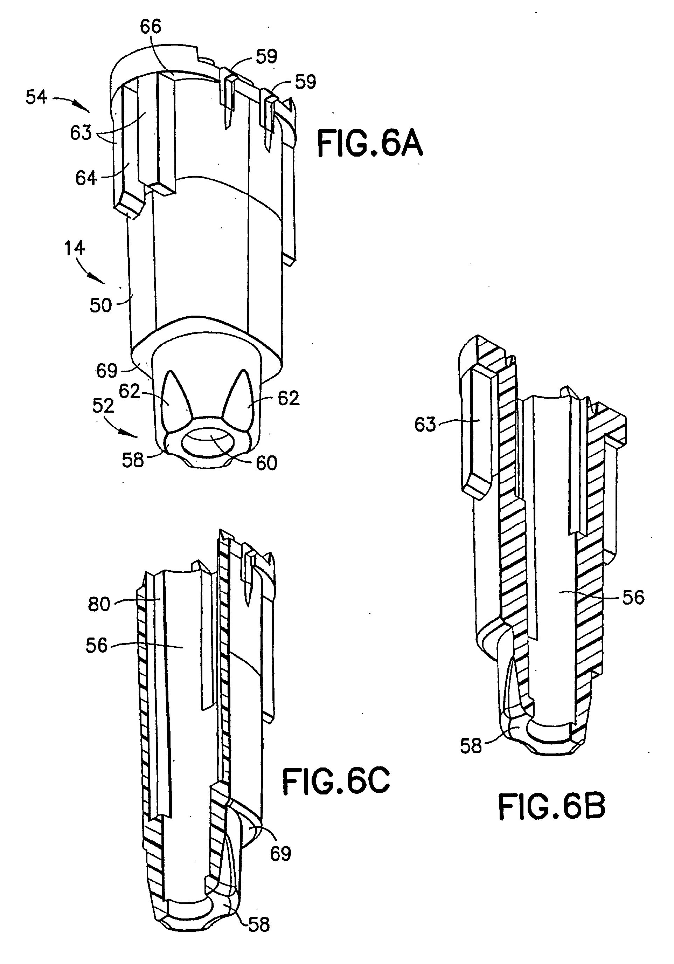

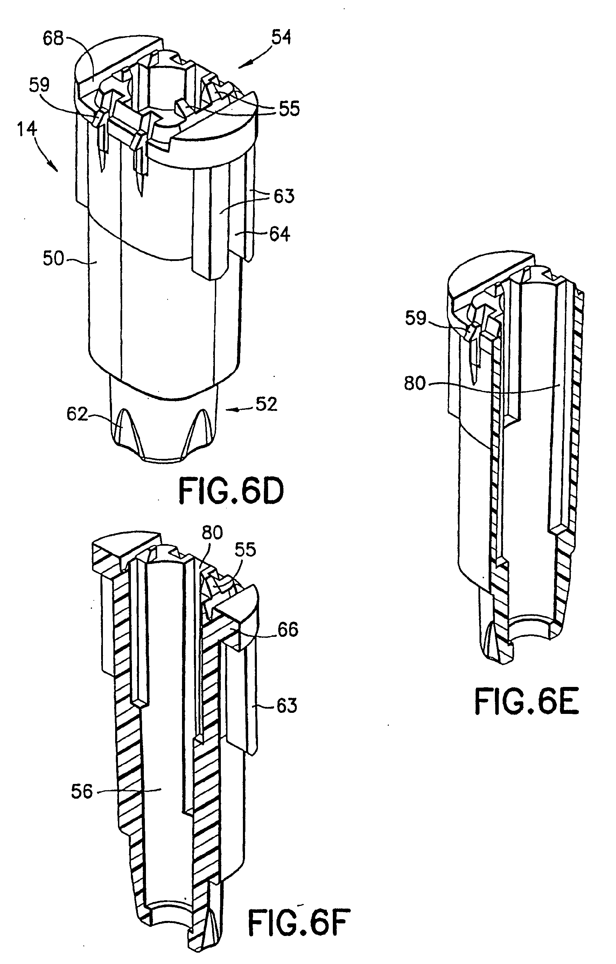

As noted above, the shield **14** extends outward from the opening **30** through the forward end of the housing **12.** As shown in FIGS. 6A-6F, the shield **14** is a generally cylindrical hollow structure defining a shield body **50** extending between a forward end **52** and a rearward end **54,** and defining an internal cavity **56** extending therethrough. The forward end **52** of the shield body **50** defines a forward end wall **58** including a forward opening **60** therethrough, through which the puncturing element extends when the lancet device **10** is actuated by the user, as will be discussed in more detail herein. The forward end wall **58** generally defines a small contact area about the distal opening **60** for contacting the intended area on the user's body which is to be punctured by the puncturing element. The reduced contact area may be made smaller (i.e., reduced in area) by providing a plurality of peripheral indentations **62** that are formed in the shield **14.** The peripheral indentations **62** may also provide a target indicia to visually aid the user in aiming the lancet device **10** generally, and aiming the puncturing element of the lancet in particular, as will be discussed in greater detail. The peripheral indentations **62** generally resemble the peripheral indentations **42** provided on the housing **12.** The peripheral indentations **42** are positioned around the perimeter of the shield **14** and may be equally spaced about the shield **14.** The peripheral indentations **42** enable the user to easily visually locate the approximate discharge point of the puncturing element, thereby improving the aiming characteristics of the lancet device **10** and ensuring optimal blood flow during a skin puncturing operation.

As noted, the shield **14** is axially or longitudinally movable within the housing **12.** The shield **14** and housing **12** may therefore include corresponding guiding surfaces for guiding the shield **14** through the housing **12.** For example, shield body **50** may include a pair of longitudinal protrusions **63** extending along an outer surface thereof, forming guide channel **64** therebetween. Housing **12** may include corresponding structure such as a guide tab **44** within the main body **20** thereof for fitting within guide channel **64.** Desirably, shield body **50** includes a pair of guide channels **64** extending longitudinally along opposing sides thereof, and housing **12** includes a pair of guide tabs **44** on opposing inner surfaces of main body **20** corresponding to each of the guide channels **64.** It is contemplated that the arrangement of the guide tabs and channels may be reversed, and other guiding surfaces may also be used. The guide tabs **44** and guide channels **64** ensure that the shield body **50** is properly aligned within housing **12,** and provide for sliding axial movement of the shield body **50** within the housing **12,** and desirably prevent or resist rotational movement. Additionally, shield body **50** may include a ledge **66** at the rearward end thereof, for interference engagement with the top surface of guide tab **44** within main body **20** of housing **12.** Alternatively or in addition thereto, shield body **50** may include a forward shoulder **69** toward the forward end thereof, and main body **20** of housing **12** may include a forward rim surface **48,** providing interference engagement therebetween. Such interferingly engaging structure prevents shield body **50** from axially sliding completely out of housing **12** through opening 30.

The housing **12** and the shield **14** may further include locking structure extending therebetween, for maintaining the shield **14** in fixed relation to the housing **12** after activation. For example, shield body **50** may include structure at the rearward end **54** for frictional engagement, or for inter-fitting engagement, with the main body **20** or rear cap **24.** For example, shield body **50** may include locking fingers **59** extending at the rearward end **54** thereof, for inter-fitting engagement with locking recesses **29** within the interior surface of rear cap **24.**

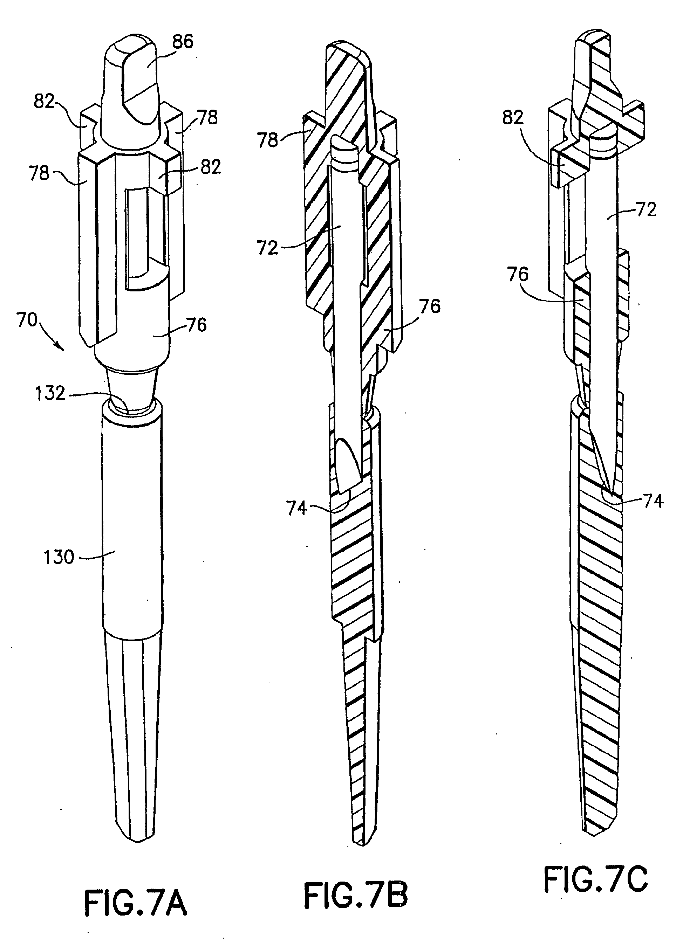

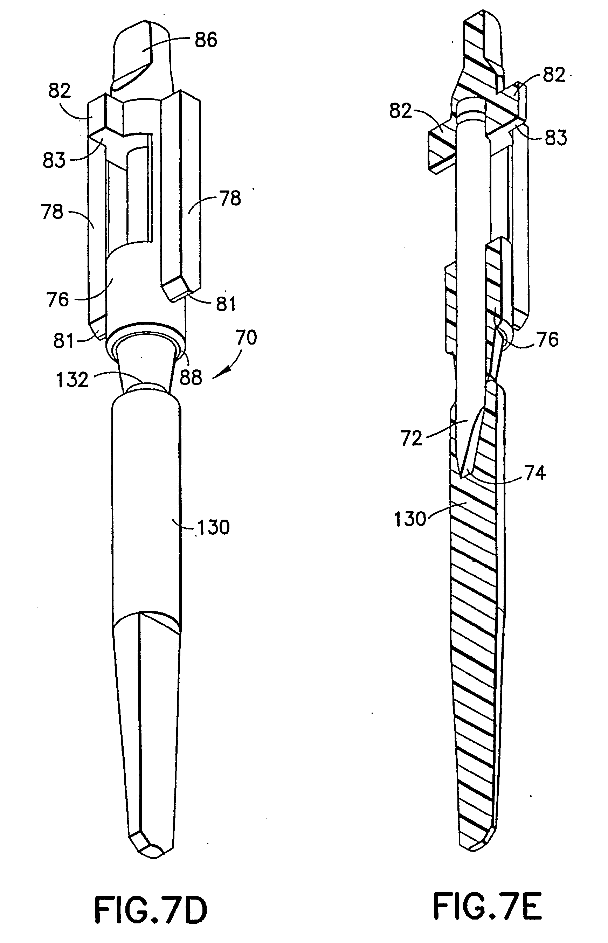

Lancet device **10** further includes a lancet structure **70** disposed within the housing **12,** and extending through shield **14.** As shown in FIGS. 7A-7F, lancet structure **70** includes a puncturing element, shown in the form of lancet **72** defining a puncturing end **74** at the forward end thereof. Lancet structure **70** is adapted for axial or longitudinal movement through the internal cavity **56** of the shield body **50** between an initial armed position with the puncturing end **74** maintained within the shield body **50** to a puncturing position in which the puncturing end **74** extends beyond the forward opening **60** of shield body **50,** as will be discussed further herein in terms of use of the lancet device **10.**

Puncturing end **74** is adapted for puncturing the skin of a patient, and may define a pointed end, a blade edge, and the like. Puncturing end **74** may include a preferred alignment orientation, such as with a pointed end of a blade aligned in a specific orientation. In such an embodiment, shield body **50** and/or main body **20** of housing may include target indicia corresponding to the alignment orientation of the puncturing end **74.** Indentations **62** of the shield body **50** and/or indentations **42** of the main body **20** may function as such an alignment orientation.

Lancet structure **70** further includes a carrier element **76** supporting lancet **72** at the rearward end thereof. The carrier element **76** and shield body **50** may include corresponding guiding surfaces for guiding the lancet structure **70** therethrough. For example, carrier element 76 may include a guide tab **78** on an external surface thereof, with the shield body **50** including a corresponding guide channel **80** extending longitudinally along an inner surface thereof for accommodating guide tab **78** slidably therein. Desirably, carrier element **76** includes a pair of guide tabs **78** on opposing lateral sides thereof, and shield body **50** includes a corresponding pair of guide channels **80** extending along opposing inner surfaces thereof corresponding to each of the guide tabs **78.** It is contemplated that the arrangement of the guide tabs and channels may be reversed, and other guiding surfaces may also be used. The guide tabs **78** and guide channels **80** ensure that the lancet structure **70** is properly aligned within shield body **50,** and provide for sliding axial movement of the lancet structure **70** within the shield body **50** and may prevent or resist rotational movement. A bottom surface **81** of the guide tabs **78** provides an abutment surface for abutting against a bottom surface of guide channels **80** to prevent the lancet structure **70** from axial movement entirely out of shield body **50** through forward opening **60.**

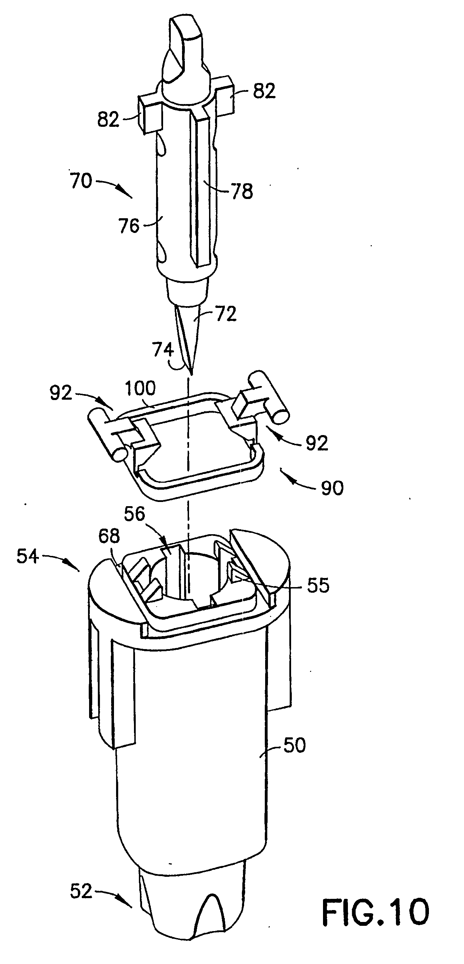

As shown in FIG. 10, retaining hub **90** is further provided, shown at the rearward end **54** of the shield body **50.** Retaining hub **90** is desirably provided as a separate structure disposed or retained within the rearward end of shield body **50.** For example, shield body **50** may include structure for accommodating retaining hub **90,** such as recess **68** extending within an upper surface of rearward end **54.** In this manner, retaining hub **90** rests within recess **68.** In other embodiments, shield body **50** may include a surface for supporting and positioning retaining hub **90** to assist in assembly. Still in other embodiments of the invention, functional elements of the retaining hub **90** may be molded or formed directly onto the shield body **50.**

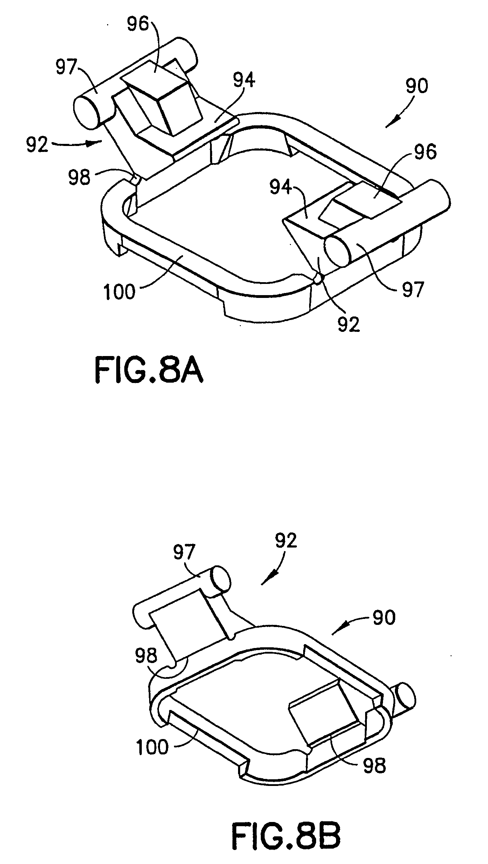

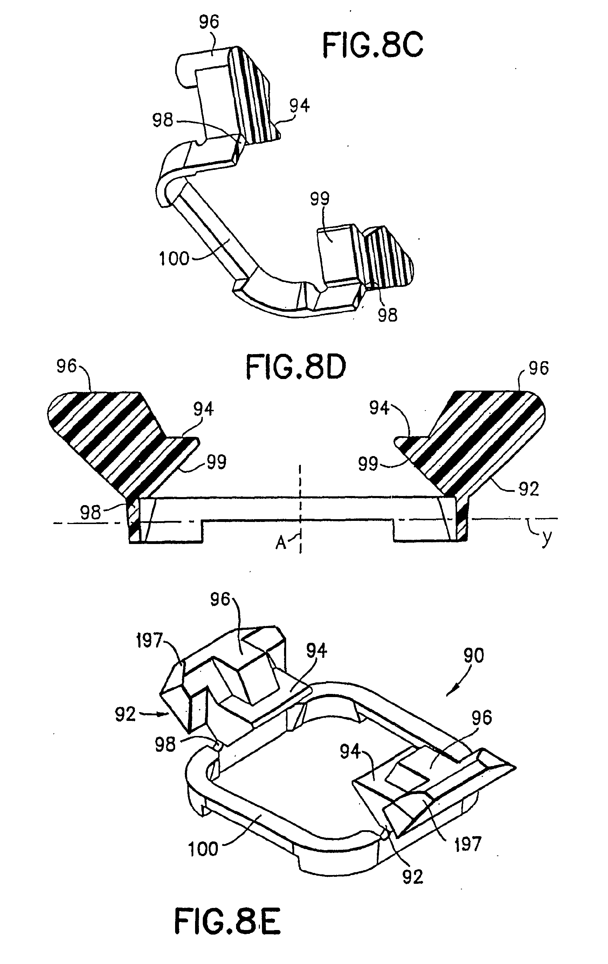

Retaining hub **90** defines a lever structure for retaining the lancet structure **70** in an initial armed position retracted within housing **12.** In particular, as shown in FIGS. 8A-8D, retaining hub **90** includes a pivotal lever element **92,** including a shoulder **94** and a contact surface **96** on the upper surface thereof, with a pivot hinge **98** on the lower surface thereof between the shoulder **94** and the contact surface **96.** As an example, lever element **92** defines a class 1 lever, in which the fulcrum or pivot point is positioned between the force and the load, as in a seesaw. For example, the upper surface of lever element **92** includes the shoulder **94** opposite the contact surface **96,** with pivot hinge **98** providing a fulcrum between the shoulder **94** and the contact surface **96.** In this manner, the load, represented by the lancet structure **70** resting on the shoulder **94,** is separated from the force, which is applied at the contact surface **96** as will be described in more detail herein, with the fulcrum of pivot hinge **98** located between the force and the load.

As depicted in FIGS. 8A-8D, lever element **92** may be provided, in one embodiment, as a pivotal generally wedge-shaped structure, with the bottom point of the wedge acting as the fulcrum at pivot hinge **98** for pivotal movement of the lever. The retaining hub **90** may include an annular rim **100,** with at least one lever element **92** supported on and pivotally hinged to the annular rim **100** through the pivot hinge **98.** Retaining hub **90** typically includes a pair of lever elements **92** pivotally hinged to the upper surface of the annular rim **100** at opposing sides thereof. Annular rim **100** is depicted herein as a generally rectangular ring-like structure with curved corner connections, extending circumferentially or perimetrically to define an interior opening. The term "annular" as used herein is intended to encompass any ring-like or band-like structure, whether circular, curved, or polygonal, including curved or angular corner connections. It is also contemplated that other annular yet incomplete rings or band-like structures may be used, such as a structure similar to a slotted or slip-on washer, which has a discontinuous annular structure.

Retaining hub **90** and lancet structure **70** are in interference engagement with each other, such that retaining hub **90** retains the lancet structure **70** in an initial armed position retracted within housing **12.** For example, carrier element **76** may include a finger **82** extending laterally therefrom, including a support surface **83** on a bottom surface of the finger **82.** Support surface **83** of finger **82** rests on shoulder **94** of lever element **92,** thereby providing interference engagement between the lancet structure **70** and the retaining hub **90.**

Moreover, contact surface **96** of lever element **92** is adapted for contacting engagement with structure within housing **12.** For example, rear cap **24** of housing **12** may include structure extending therein, such as internal contact **46** integrally formed and extending on at least one, and desirably on two opposing inner sidewalls thereof. Each internal contact **46** includes an engagement surface **47** for contacting engagement with contact surface **96** of lever element **92,** forming a cam surface. In one embodiment, contact surface **96** includes a generally rod-shaped portion **97,** and the internal surface of rear cap **24** includes a pair of internal contacts **46** extending adjacent each other on the inner wall surface, and on each opposing side of the inner wall surface. In this manner, the pair of internal contacts **46** engages opposing ends of the rod shaped portion **97** of contact surface **96,** thereby providing a continual cam-like contact surface around the perimeter of the rod shaped portion **97** during pivotal movement of lever element **92.** In an alternate embodiment shown in FIG. 8E, contact surface **96** may include angular surfaces **197** forming a chamfered portion as opposed to the generally rod-shaped portion discussed above. Such a chamfered portion formed by angular surfaces **197** is particularly useful in molding operations for forming of the retaining hub **90** in conventional injection molding procedures. With such an embodiment, the pair of internal contacts **46** within rear cap **24** engages opposing ends of the chamfered portion formed by the angular surfaces **197** of contact surface **96,** providing for a cam-like contact surface as noted above.

Moreover, lever element **92** is typically positioned on one side of a plane, shown in FIG. 8D at plane **Y,** dissecting the annular rim **100** at a cross section to the general longitudinal axis A which generally defines the lancet device **10** and the direction of travel of lancet structure **70.** In this manner, the fulcrum, such as that defined through pivot hinge **98,** represents a low area moment of inertia above the plane **Y,** such as at the top surface of annular rim **100,** to cause plastic deformation of lever element **92,** and namely pivot hinge **98,** as the lever element **92** pivots outwardly. Such plastic deformation may be permanent, such that after the lever elements pivot, they maintain their shape and position.

Movement of the lancet structure **70** through the lancet device **10** is achieved through a biasing force provided through a drive spring **102.** Drive spring **102** is adapted to exert a biasing force against lancet structure **70** to drive lancet structure **70** through the device toward the puncturing position, and may be disposed between the rearward end of the housing **12** and the lancet structure **70.** Drive spring **102** may be a separate element contained between the rearward end of housing **12** and the lancet structure **70,** or may be integrally formed with one or both of housing **12** and/or lancet structure **70.** Rear cap **24** may include structure for alignment of and/or for maintaining drive spring **102** in the proper orientation. For example rear cap **24** may include an alignment nub **104** for accommodating the drive spring **102.** The lancet structure **70** may also include a surface or structure for accommodating an opposing end of the drive spring **102,** such as a rear nub **86** extending from the carrier element **76** of lancet structure **70.** Drive spring **102** extends between alignment nub **104** of rear cap **24** and rear nub **86** of carrier element **76.** When the lancet structure **70** is in an armed position, the drive spring **102** exerts a force against the lancet structure, such as between the rearward end of housing **12** and the lancet structure **70,** biasing the lancet structure **70** toward the puncturing position. The shield body **50** and lever element **92** may include inter-engaging structure to prevent lever element **92** from pivoting in a reverse direction about pivot hinge **98,** regardless of the biasing force applied against lancet structure **70** and shoulder **94** through drive spring **102.** For example, bottom angled surfaces **99** formed from the wedge-shaped lever element **92** may engage and abut corresponding angled nibs **55** on the rearward end **54** of shield body **50.** Such inter-engaging surfaces prevent any applied force from drive spring **102** from pivoting the lever element **92** about pivot hinge **98** in a reverse direction, that is in a direction such that shoulder **94** pivots downwardly into interior cavity **56** of shield body **50.** Optionally or in addition thereto, the plastic deformation of pivot hinge **98** as discussed above may be permanent, thereby preventing lever element **92** from automatically pivoting in a reverse direction to enable the lancet structure **70** to be re-set in a pre-actuation state resting on shoulder **94** after actuation.

A retraction spring **110** may further be provided at the forward end of the lancet device **10,** for retracting the lancet structure **70** within the shield body **50** after the lancet structure **70** is axially moved to the puncturing position. Retraction spring **110** typically extends between a forward surface **88** of the carrier element **76** of lancet structure **70** and an inner surface within the forward end wall **58** of the shield body **50.** Retraction spring **110** is typically a compression spring, capable of storing energy when in a compressed state.

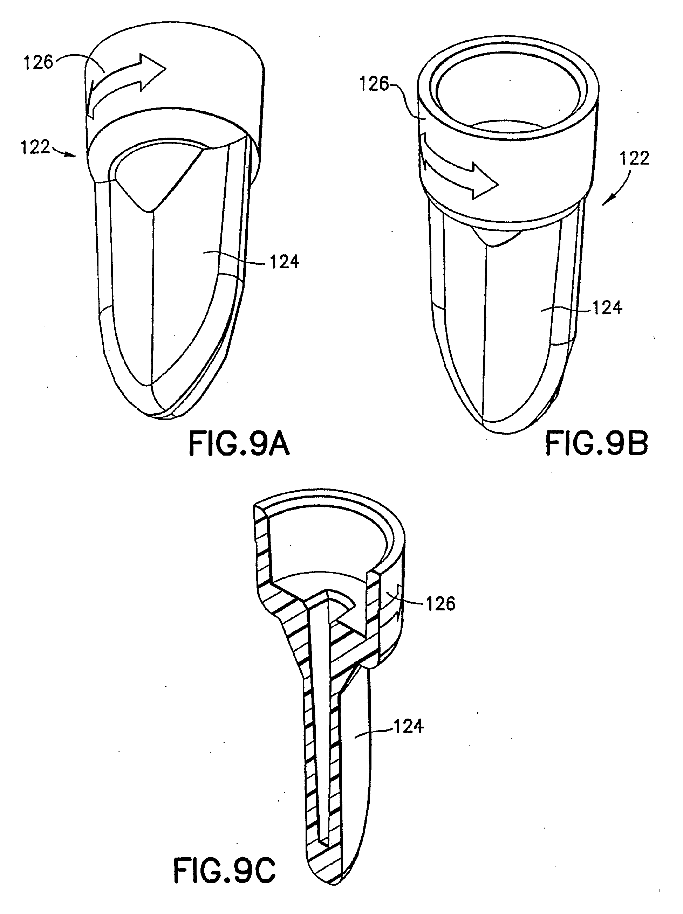

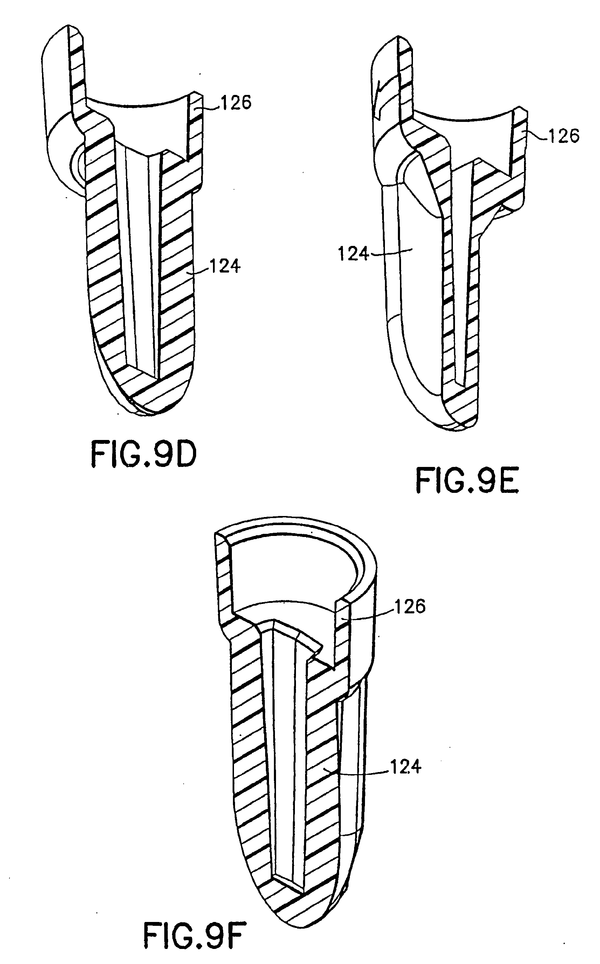

Lancet device **10** may further include a protective cover **120** for protectively covering the lancet device **10** prior to use thereof. The protective cover **120** may include a tab member **122** associated with the forward end of the lancet device **10,** which maintains sterility of the forward end wall **58** of shield body **50.** Referring to FIGS. 9A-9F, tab member **122** may include a forward tab portion **124** and a depending skirt **126.** The depending skirt **126** is adapted to cooperate with the forward end **52** of the shield body **50,** generally encompassing or enclosing the forward end **52.** The depending skirt **126** also contacts the forward end **22** of the main body **20** of the housing **12.** In this manner, the tab member **122** encloses forward opening **30** of main body **20** and forward opening **60** of shield body **50.** Moreover, such arrangement maintains the respective forward ends of main body **20** and shield body **50** in fixed relation with respect to each other, thereby preventing movement therebetween which could cause premature activation of the lancet device **10.**

A portion of the protective cover **120** may extend within the shield body **50** to encompass at least a portion of the puncturing element. For example, as shown in FIG. 11D, a post portion **130** extends through forward opening **60** of shield body **50** and into internal cavity **56** thereof, protectively surrounding and encompassing at least a portion of the puncturing element, namely lancet **72.** The post portion **130** and tab member **122** may be separate elements which are affixed or otherwise maintained together. For example, tab member **122** may include an inner opening for accommodating post portion **130** therethrough. Referring generally to FIGS. 7A-7E, post portion **130** may be formed integrally with carrier element **76** of lancet structure **70,** completely encompassing lancet **72,** thereby maintaining sterility thereof prior to use. Post portion **130** and carrier element **76** may include a notched portion **132** at a juncture therebetween, providing a fraction point for removing post portion **130** and exposing lancet **72.** Alternatively, the post portion **130** may be secured directly to the lancet **72** by methods customary in the medical field, such as with a releasable medical grade adhesive.

In one embodiment, the rear cap **24** and the housing body **20** are separate structures which are mated, with the housing body **20** forming a forward portion of the housing **12** and the rear cap **24** forming a substantial rear portion of the housing **12.** More particularly, the rear cap **24** may constitute a significant portion of the housing **12,** such as approximately half of the housing **12,** mating with the housing body **20** at a location dividing housing **12** approximately in half when measured by the complete longitudinal length of the housing and rear cap together. Such an arrangement provides for simplified assembly of the lancet device **10,** in that the internal components including shield **14,** lancet structure **70,** and the retaining and engaging elements including retaining hub **90,** drive spring **102** and optionally retraction spring **110,** may be inserted within the housing body **20** from the rearward end thereof, requiring little clearance provided by the small size of housing body **20** for insertion. Additionally, after inserted as such, such internal elements may be easily seen due to the low clearance provided by the relative small size housing body **20** with respect to the overall housing **12,** thereby permitting easy visual assurance of proper alignment. Moreover, the rear cap **24** may then be fitted to housing body **20** at a location adjacent the internal functional components, such as at a location substantially peripheral to the retaining hub **90** within the housing **12.** Also, the housing body **20** and the rear cap **24** may mate at a substantial midpoint of the total length of the housing **12,** with each substantially defining a half portion of the housing **12.** In this manner, the mating of the housing body **20** and the rear cap **24** substantially intersects or bisects the finger grip indentations **37, 38.**

The respective elements of the lancet device of the present invention are all typically formed of molded plastic material, such as a medical grade plastic material. The lancet 72 may be constructed of any suitable material adapted for puncturing the skin, and is typically a surgical grade metal such as stainless steel.

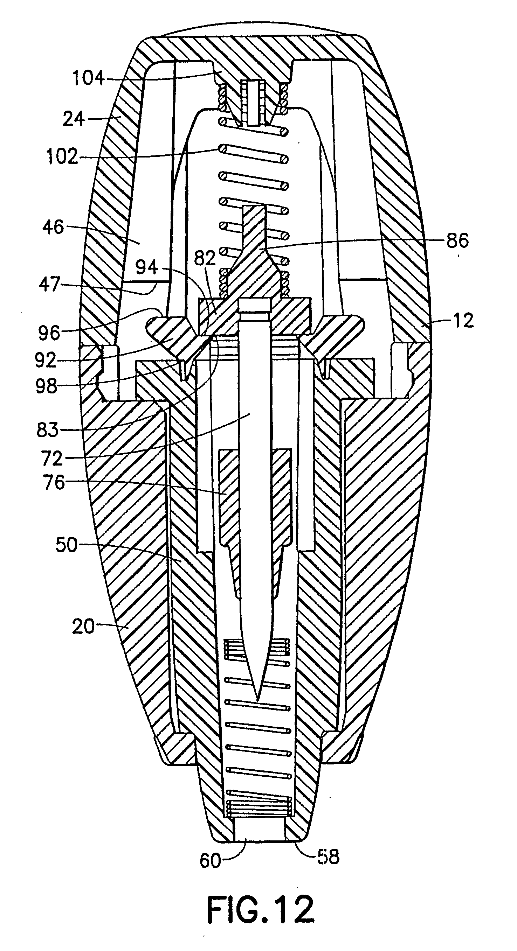

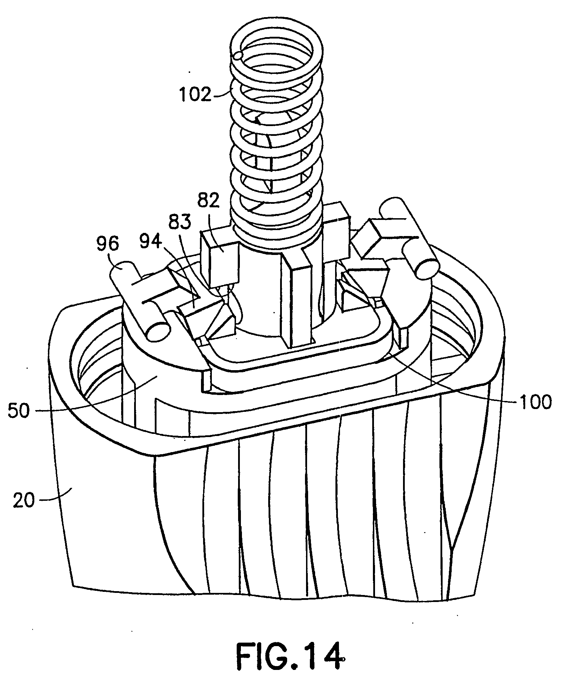

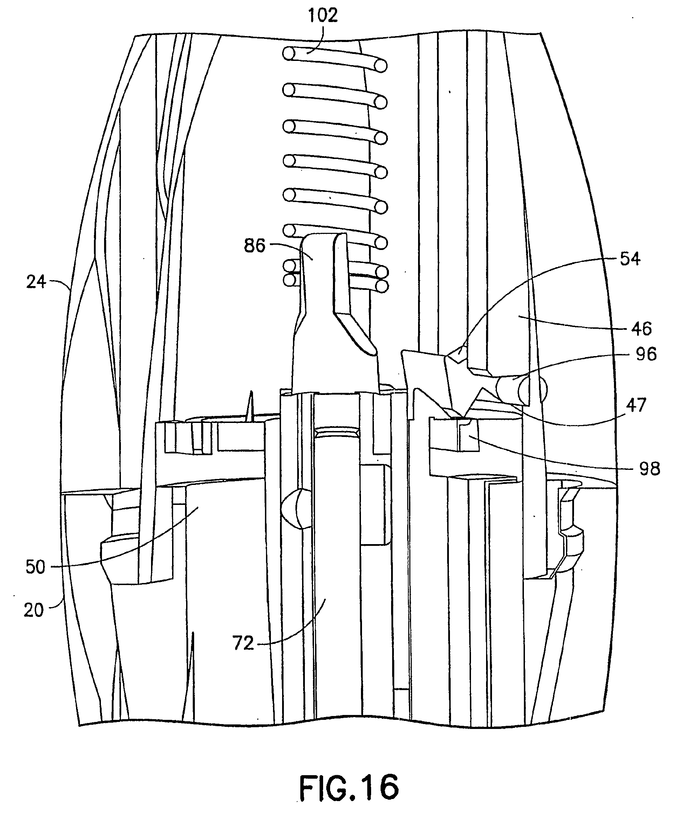

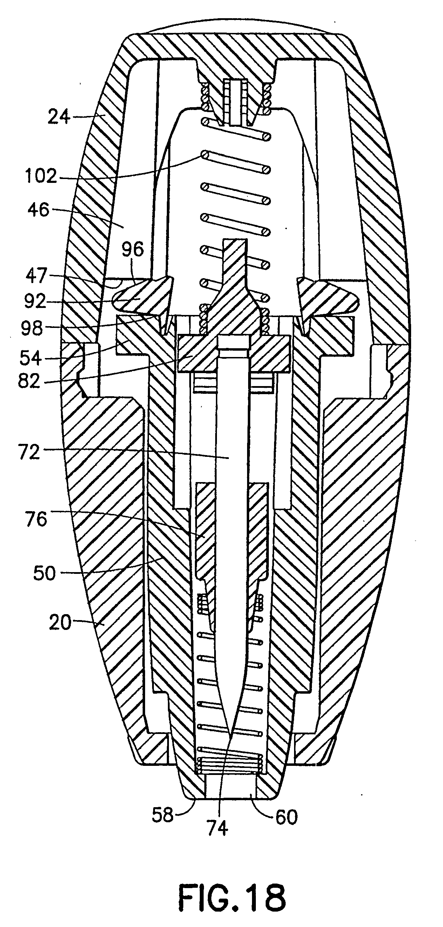

Use of the lancet device **10** will now be described with general reference to FIGS. 1-18, and particular reference to FIG. 11D and FIGS. 12-18. Prior to use, lancet device **10** is provided as shown in FIGS. 1 and 11D, with protective cover **120** covering shield **14** at the forward end thereof. Lancet device **10,** and in particular lancet structure **70,** is in an initial pre-actuation state, with finger **82** of carrier element **76** abutting or resting upon shoulder **94** of the lever element **92** in interference engagement therewith. In this manner, lever element **92** of the retaining hub **90** maintains lancet structure **70** in this pre-actuation position within housing **12,** and in particular with puncturing end **74** maintained retracted within shield body **50.** Further, drive spring **102** extends between the lancet structure **70** and the rear cap **24** of housing **12.** In this pre-actuation position, drive spring **102** may be in a relaxed state or may be in a fully compressed state. More desirably, drive spring **102** is in a partially compressed state in this pre-actuation position, exerting a biasing force between rear cap **24** and lancet structure **70,** with the interference engagement between finger **82** and shoulder **94** maintaining lancet structure **70** against any such biasing force. Moreover, the inter-engaging surfaces between bottom angled surfaces **99** and angled nibs **55** prevent lever element **92** from pivoting in a reverse direction, thereby forcing lancet structure **76** through shield body **50.** Also, in this state, protective cover **120** prevents any axial movement of shield **14** with respect to housing **12,** thereby preventing actuation of the lancet device **10.**

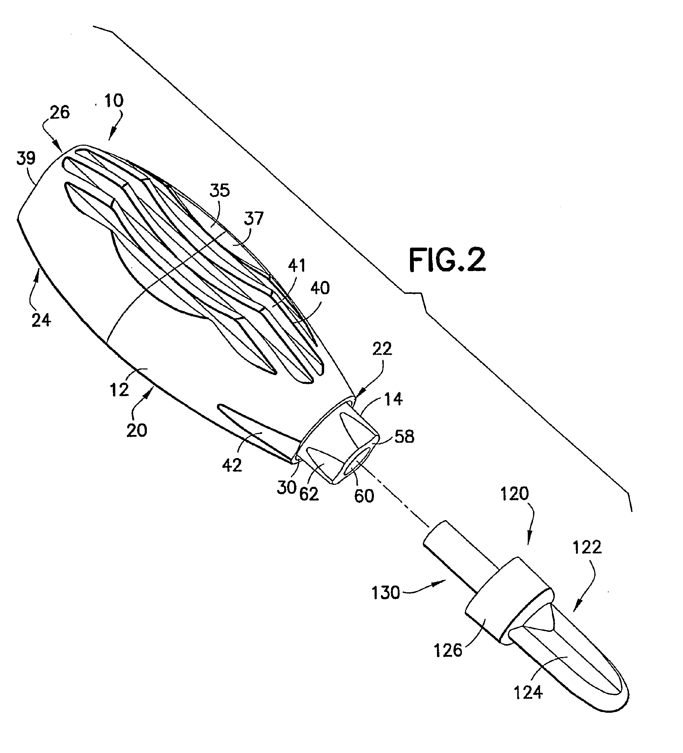

To prepare the lancet assembly for use, the user grasps housing **12,** such as between a finger and thumb on opposing sides **35, 36,** and removes the protective cover **120** from the forward end as shown in FIG. 2, thereby exposing the shield body **50** extending from the forward end of main body **20** of housing **12.** The forward tab portion **124** of the tab member **122** may be ergonomically formed, such as through the inclusion of a paddle-shaped member, to allow the user to easily manipulate the tab member **122** and apply the necessary force or torque to release the depending skirt from frictional engagement with the forward end of the shield body **50,** and to break the post portion **130** from the carrier element **76** at the notch **132** to thereby release the post portion **130** from the lancet **72.** The applied breaking force is in accordance with the present invention and may be a singular twisting or pulling motion, or a combined "twisting" (i.e. rotational) and "pulling" motion applied for breaking the connection between the post portion **130** and the carrier element **76,** as well as to release the frictional engagement between the depending skirt **126** and the shield body **50.**

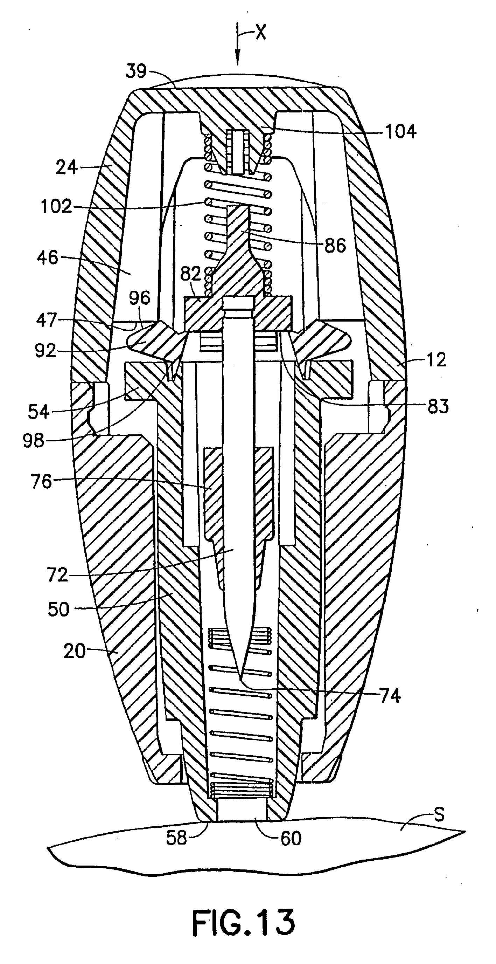

The forward end wall **58** of shield body **50** may then be contacted with a location on the user's body or another person's body where it is desired to initiate blood flow, such as the patient's skin surface **S** as shown in FIG. 13. If provided, target indicia, such as indentations **62,** may be aligned with the desired location of puncture.

Once placed against the body, the user exerts a downwardly directed force on the housing **12** forcing shield body **50** against skin surface S. In particular, the user applies a force against the finger grip indentation **39** of the rear cap **24** in the direction of Arrow **X,** thereby applying a force against the skin surface **S.** Such force establishes an opposing external pressure force between the forward end wall **58** of the shield body **50** and the rear cap **24** of the housing **12** causing the shield body **50** to move axially within the housing **12,** thereby displacing the rearward end **54** of the shield body toward the rear cap **24.** The corresponding guiding surfaces provided through guide tabs **44** and guide channels **64** guide the shield body **50** axially through the main body **20** of housing **12,** ensuring proper axial alignment therebetween.

Since retaining hub **90** is adjacent rearward end **54** of shield body **50,** such displacement of the rearward end **54** of the shield body toward the rear cap **24** causes corresponding rearward movement of retaining hub **90** toward rear cap **24.** Moreover, the interference engagement between shoulder **94** of lever element **92** of retaining hub **90** and finger **82** of carrier element **76** of lancet structure **70** causes corresponding rearward movement of lancet structure **70** toward the rear cap **24.** Such movement causes drive spring **102** to compress. In embodiments in which drive spring **102** is in a relaxed state in the initial pre-actuated position, this compressing of drive spring **102** arms drive spring **102** with a biasing force sufficient to propel lancet structure **70** axially forward through shield body **50** to the puncturing position, thereby providing lancet structure **70** in an armed position. At this point, however, lancet structure **70** is still maintained such that puncturing end **74** is retracted within shield body **50** due to the interference engagement between finger **82** and shoulder **94.** In embodiments in which drive spring **102** is in a partially compressed state in the initial pre-actuated position, this compressing of drive spring **102** further arms drives spring **102** with additional biasing potential energy sufficient to fully propel lancet structure **70** axially forward through shield body **50** to the puncturing position. Again, in this pre-actuated armed position, lancet structure **70** is still maintained such that puncturing end **74** is retracted within shield body **50** based on the interference engagement between finger **82** and shoulder **94.**

During such axial or longitudinal movement of shield body **50** toward rear cap **24,** the retaining hub **90** is also displaced rearwardly (or proximally) toward rear cap **24,** with fingers **82** of the carrier element **76** resting upon shoulders **94** of the lever elements **92.** As shown in FIGS. 13-14, such rearward movement of retaining hub **90** causes the cam surfaces of engagement surfaces **47** of the internal contacts **46** within rear cap **24** to engage and co-act with the corresponding contact surfaces **96** of lever elements **92,** such as the rod shaped portions **97.** Accordingly, the corresponding camming contact surfaces provide an actuator element for the lancet device **10.** Such engagement and co-action causes the lever elements **92** to pivot about pivot hinges **98** with respect to annular rim **100** due to the wedge-shaped profile of the lever elements **92.** In particular, with the shoulders **94** extending generally radially inwardly of the annular rim **100** and the contact surfaces **96** generally on an external perimeter of the annular rim **100,** engagement surfaces **47** engage the contacting surfaces **96,** and in particular the rod shaped portions **97,** at an external perimeter of the annular rim **100,** thereby pivoting the lever element **92** about the fulcrum of pivot hinge 98 by tipping the contact surfaces **96** and the shoulders **94** to release the lancet structure through the annular rim **100** and into the internal cavity **56** of the shield body **50.**

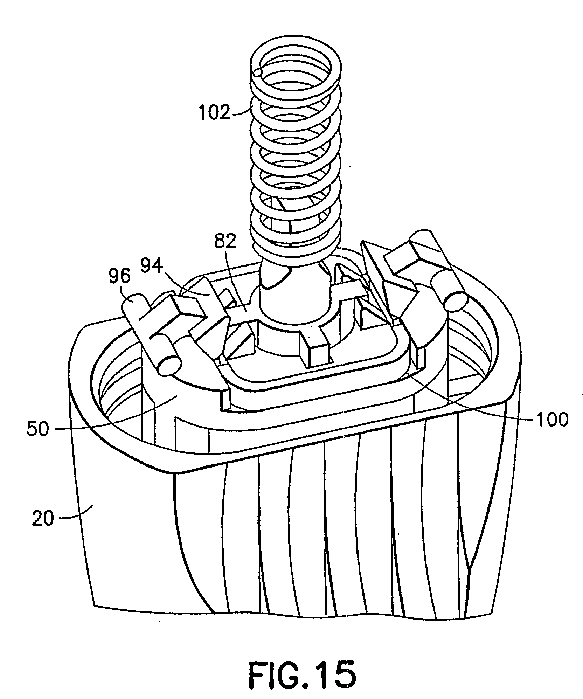

Such engagement provides for actuation of the lancet device. In particular, the pivoting of lever elements **92** about pivot hinges **98** further displaces shoulders **94** toward the rearward end of rear cap **24,** thereby further compressing and further biasing drive spring **102.** Continued axial displacement of shield body **50** toward rear cap **24** causes further engagement of the corresponding surfaces of internal contacts **46** and contact surfaces **96,** such that engagement surfaces **47** cam or ride about the perimeter of rod-shaped portions **97,** thereby further pivoting lever elements **92.** Eventually, such pivoting causes shoulders **94** to be pivoted to a point at which the interference engagement between shoulders **94** and fingers **82** of carrier element **76** is released, as shown in FIGS. 15-16. At this point, fingers **82** are free from shoulders **94** and may axially move through the internal opening through annular rim **100.** The biasing force of drive spring **102** propels lancet structure **70** downward away from the rear cap **24** axially through housing **12** and shield body **50.** During such movement, corresponding guide tabs **78** and guide channels **80** guide lancet structure **70** axially through shield body **50.** Moreover, shield body **50** may further include additional channels in alignment with and adapted for accommodating fingers **82** of lancet structure **70** in sliding relation during such axial movement therethrough.

Actuation of the lancet device **10** is therefore achieved through the interfering or camming engagement contact between the engagement surfaces **47** and contact surfaces **96,** providing the pivoting movement of lever element **92.** As noted, such pivoting movement results in both compression of drive spring **102** to arm or to further arm the lancet structure **70** and sequential release of the interference engagement maintaining the lancet structure **70** in the pre-actuated or armed position. Accordingly, actuation of the lancet device **10** achieves sequential arming and release of the lancet structure through a single motion of the device. Moreover, such sequential arming and release merely requires movement of the inter-engaging contact surfaces between the housing **12** and the pivoting lever element **92.** It is therefore contemplated that such sequential arming and release may be attained regardless of whether an axially moveable shield is included, so long as some mechanism for movement of the inter-engaging surfaces with respect to each other is provided.

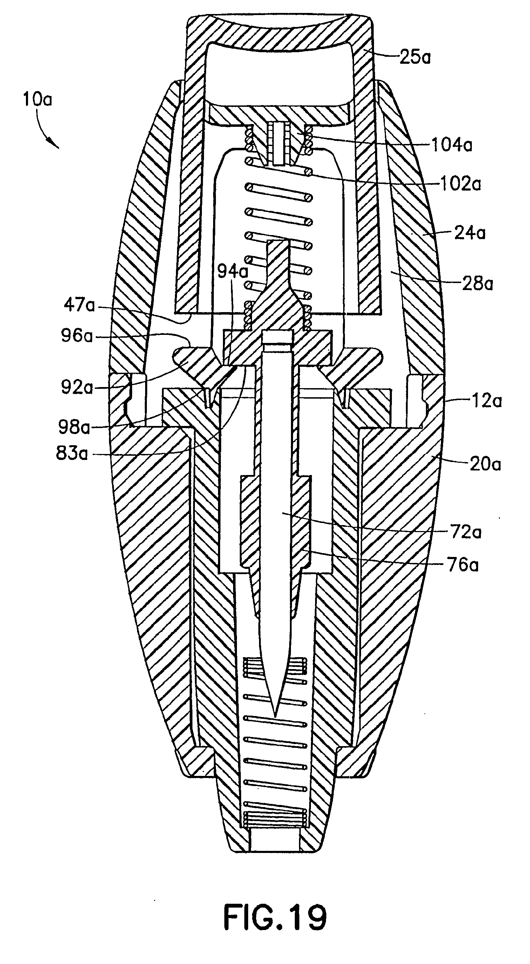

For example, FIG. 19 depicts a cross-sectional view of a lancet device **10a** in an alternate embodiment of the present invention. In this embodiment, actuation is achieved through an actuator including an actuation element, such as push button **25a.** In particular, housing **12a** is defined by main body **20a** and rear cap **24a.** Push button **25a** extends through housing **12a** at rear cap **24a,** and into the internal cavity **28a** therein. Actuation of lancet device **10a** is accomplished by axially moving push button **25a** within housing **12a,** such that one or more engagement surfaces **47a** at the forward end of push button **25a** within housing **12a** contact the corresponding contact surface(s) **96a** of lever element **92a,** thereby pivoting the lever element **92a** about pivot hinge **98a.** As in the aforementioned embodiment, such contacting and pivoting releases the interference engagement between the support surface **83a** of carrier element **76a** and the shoulder **94a** of lever element **92a,** thereby permitting drive spring **102a** to propel lancet **72a** through housing **12a** to the puncturing position. Drive spring may be maintained between carrier element **76a** and push button **25a** through alignment nub **104a** within push button **25a.** Alternatively, the drive spring may be maintained between the carrier element and the rearward end of the housing, with the push button element extending through the housing to cause pivotal actuation.

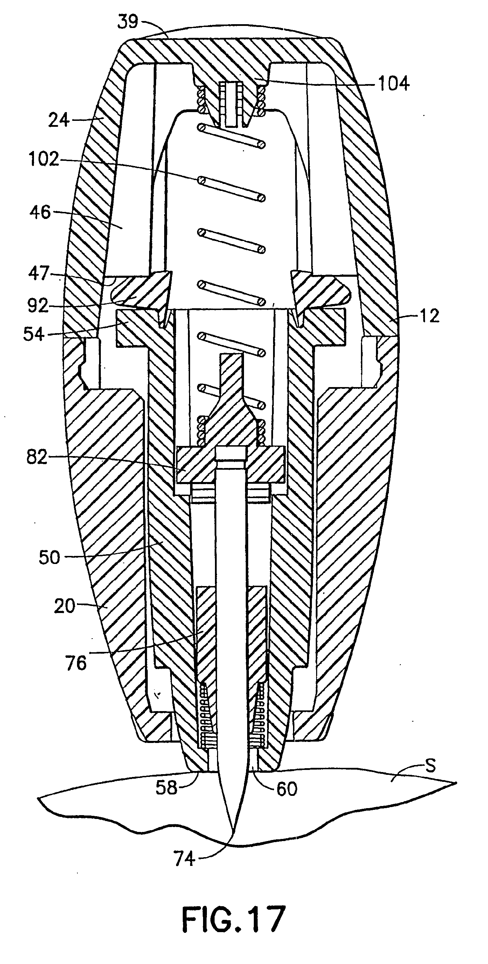

Returning to the actuation as shown in FIG. 17, the biasing force of drive spring **102** propels the lancet structure **70** through shield body **50** to a puncturing position, in which puncturing end **74** of lancet **72** extends through the forward opening **60** through forward end wall **58** a sufficient distance to permit the puncturing end **74** to puncture the skin surface **S.** The bottom surface **81** of the guide channels **80** within shield body **50** provides an abutment surface for guide tab **78** to prevent the lancet structure **70** from axial movement entirely out of shield body **50** through forward opening **60** during such propelling. Moreover, during such propelling, the forward surface **88** of carrier element **76** contacts the rearward end of retraction spring **110,** which is maintained within the forward end **52** of shield body **50,** desirably in a relaxed condition in the pre-actuated and/or armed state of the lancet device. The propelling force from the bias of drive spring **102** causes such contact with retraction spring **110,** thereby compressing retraction spring **110** between the forward surface **88** of lancet structure **70** and the interior of the forward end wall **58** of shield body **50.** The structure of retraction spring **110** is designed such that it is compressible, based upon the biasing force of drive spring **102** propelling lancet structure **70,** to permit puncturing end **74** of lancet **72** to extend through forward opening **60.** Moreover, the retraction spring **110** is a compression spring, and is therefore capable of being compressed in this manner, but includes sufficient resiliency to return to a relaxed condition after the lancet structure **70** extends to the puncturing position. Accordingly, the biasing force of the compression spring **110** between the forward end wall **58** of the shield body **50** and the lancet structure **70** when in a relaxed state exceeds the biasing force of the drive spring **102** acting between the rear cap **24** of the housing **12** and the rear nub **86** of the lancet structure **70** after the drive spring drives the lancet structure **70** to the puncturing position. In this manner, the retraction spring **110** will relax to an uncompressed state, thus applying a biasing force between the forward surface **88** of the lancet structure **70** and the interior surface of the forward end wall **58,** thereby forcing the lancet structure **70** rearward toward the rear cap **24.** Such biasing force retracts the puncturing end **74** of lancet **72** within the shield body **50** to a position in which it is shielded from exposure through forward opening **60.** Moreover, the opposing forces acting between the drive spring **102** and the retraction spring **110,** and the respective forces of such springs based on the structure thereof, maintains the lancet structure **70** disposed within the housing **12** with puncturing end **74** shielded within shield body **50,** preventing further movement of lancet structure **70** to the puncturing position.

Moreover, after activation of the lancet device, that is, after the lancet structure **70** is retracted within the housing **12** after the puncturing position, the shield body **60** and the housing **12** may be locked in a fixed relation. In particular, with shield body **50** axially displaced toward the rear cap **24,** locking fingers **59** may deflect and lock within respective recesses **29,** thereby locking shield body **50** in a rearward position with respect to rear cap **24** and housing **12.** The lancet device **10** is therefore safely protected from re-use and may be properly discarded, such as in an appropriate medical waste container.

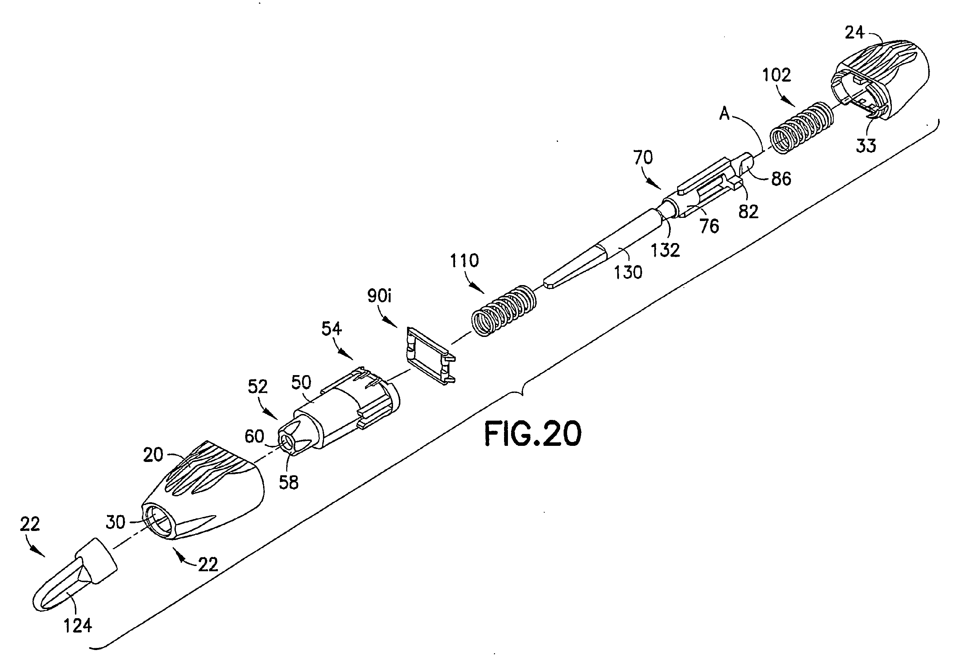

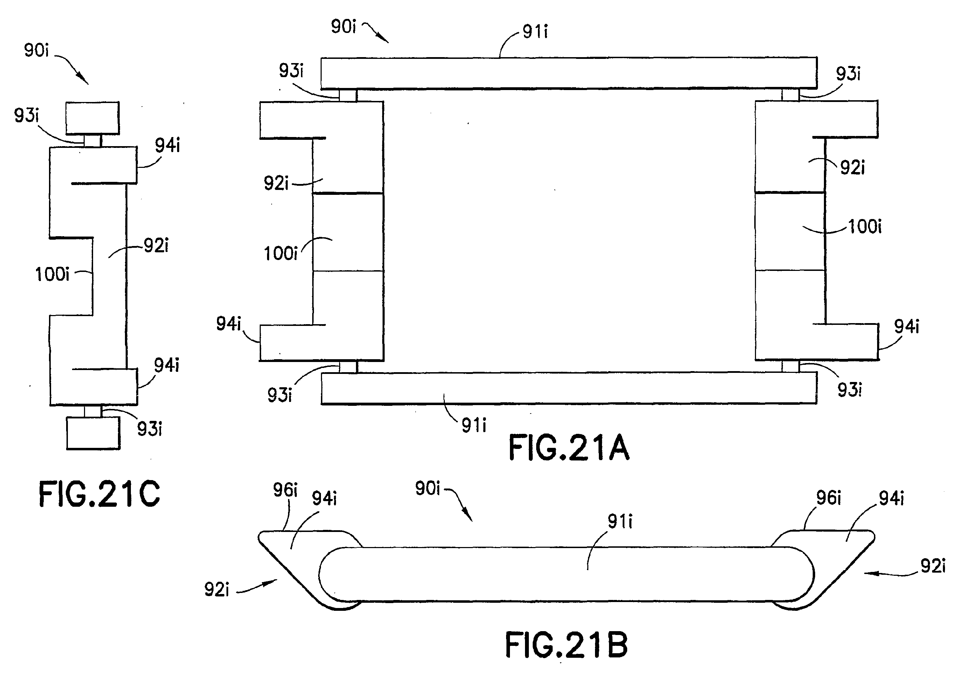

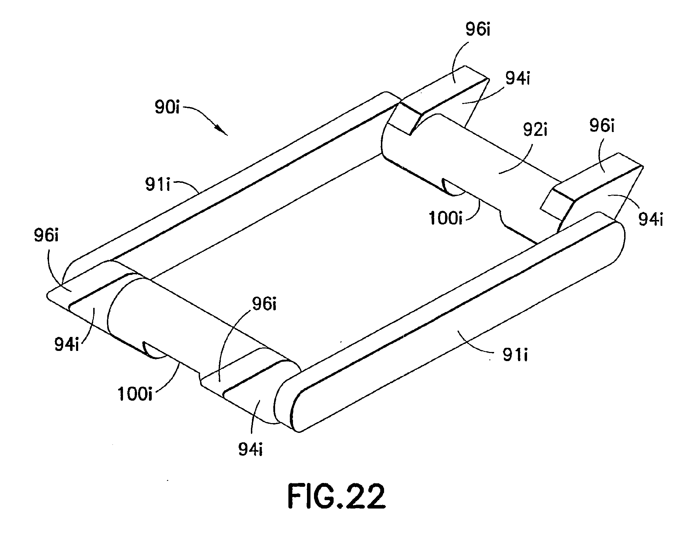

Referring to FIGS. 20-22, the lancet device may include a modified version of a retaining hub **90i.** FIG. 20 shows the retaining hub **90i** as part of the lancet device **10** as disclosed above, with similar reference numbers shown in FIGS. 20-22 referring to similar elements described in connection with FIGS. 1-18. Retaining hub **90i** generally defines an annular shape and is adapted to maintain the lancet structure **70** in an initial armed position retracted within the housing defined by main body **20** and rear cap **24.** Retaining hub **90i** typically includes two opposed and elongated support members **91i** connected by two pivotal cam elements **92i** to form the annular shape of retaining hub **90i.** Cam elements **92i** each include two outward-extending shafts **93i** engaged pivotally with the opposed support members **91i.** Cam elements **92i** each further include at least one typically wedge-shaped contact element **94i** defining an upper contact surface **96i** on the upper surface thereof. Cam elements **92i** each further define a generally centrally located recess or cut-out **100i** defined in a bottom side thereof. The purpose of recess **100i** is described herein in connection with the operation of retaining hub **90i** in lancet device **10.** As shown in FIGS. 21A and 22, the cam elements **92i** desirably each include two contact elements **94i** disposed generally at opposite ends of the cam elements **92i,** with the recess **100i** defined in the bottom side of the cam elements **92i** between the contact elements **94i.**

In the lancet device of this embodiment, retaining hub **90i** and lancet structure **70** are in interference engagement with each other, such that retaining hub **90i** retains the lancet structure **70** in an initial armed state retracted within the housing. For example, fingers **82** on carrier element **76** may rest on the upper side of cam elements **92i,** thereby providing interference engagement between the lancet structure **70** and the retaining hub **90i.** Moreover, upper contact surface **96i** on the contact elements **94i** may be adapted for contacting engagement with structure within the housing. For example, rear cap **24** may include structure extending therein, such as internal contact **46** integrally formed and extending on at least one, and desirably on two opposing inner sidewalls thereof. As retaining hub **90i** typically includes two contact elements **94i** on each cam element **92i,** two internal contacts such as contacts **46** described above may be provided on each of the two opposing inner sidewalls of the housing. Each internal contact includes a distal engagement cam surface such as cam surface **47** described above for contacting engagement with the corresponding contact surface **96i** on contact elements **94i.**

During usual operation of the lancet device of FIGS. 20-22, axial or longitudinal movement of shield body **50** toward rear cap **24,** causes the retaining hub **90i** to be displaced rearwardly toward rear cap **24,** with fingers **82** of the carrier element **76** resting upon the cam elements **92i.** Such rearward movement of retaining hub **90i** causes the contact surfaces of the engagement cam surfaces of the internal contacts within rear cap **24** to engage and co-act with the corresponding contact surfaces **96i** on the contact elements **94i** of cam elements **92i.** Such engagement and continued downward or distal movement of the internal contacts causes the cam elements **92i** to pivot on or rotate about shafts **93i** with respect to support members **91i.** Due to the generally wedge-shaped profile of the contact elements **94i,** the pivotal movement of cam elements **92i** has the effect of further compressing drive spring **102** by further "lifting" fingers **82,** at least until the point where rear nub **86** on carrier element **76** contacts the inner side of rear cap **24.** At this point, continued axial or longitudinal displacement of shield body 50 toward rear cap **24** pivots cam elements **92i** to a position where recess **100i** defined in the bottom side of cam elements **92i** has rotated to a position generally aligned with fingers **82** at which point the interference engagement between fingers **82** and cam elements **92i** is released by such alignment. The biasing force of drive spring **102** then propels lancet structure **70** downward away from the rear cap **24** axially through the housing and through shield body **50** axially through the annular opening defined by retaining hub **90i.**

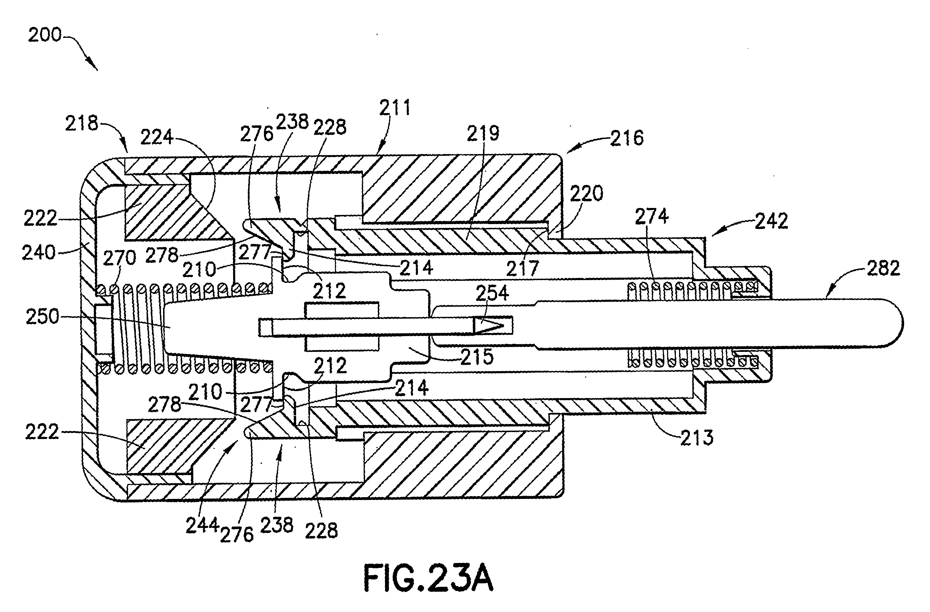

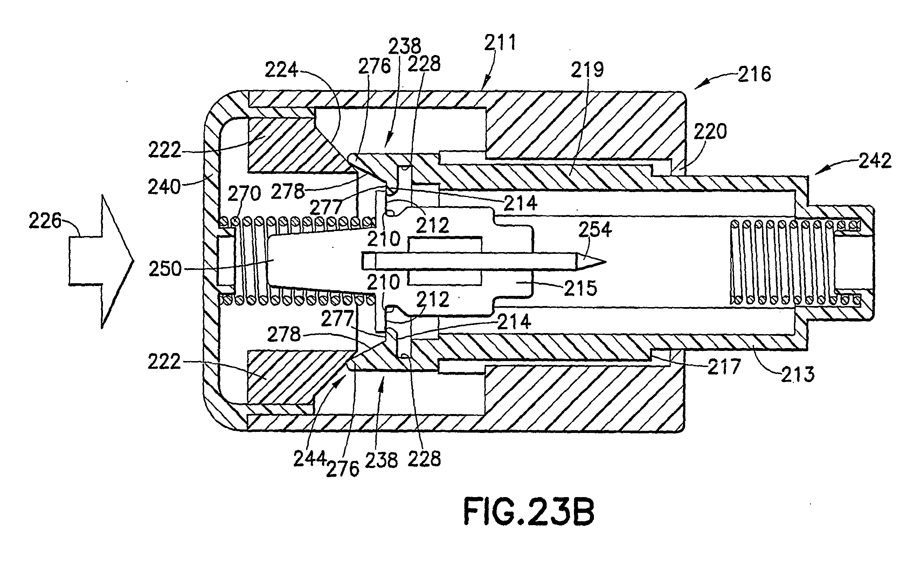

Referring to FIGS. 23A-23D, a further variation or modification of the lancet device is generally illustrated in a further embodiment. In the embodiment of FIGS. 23A-23D, a puncturing device in the form of lancet device **200** is shown. The lancet device **200** generally includes a housing **211,** a shield **213** received partially within and axially movable relative to the housing **211,** and a skin puncturing assembly **215** (which may be similar to the lancet structure **70** discussed above) disposed within the housing **211.** The housing **211** is preferably a generally tubular structure having a distal end **216** and a proximal end **218,** and may include similar structure to the housing **12** discussed above in connection with FIGS. 1-18 including a main body **20** and a rear cap **24.** Desirably, the housing **211** is open-ended at the distal and proximal ends **216, 218.** An end cap **240** may be provided at the proximal end **216** of the housing **211** to close the proximal end **218** of the housing **211.** Alternatively, the housing **211** may be formed to have a closed proximal end **218** instead of the end cap **240.** In such an embodiment, the closed proximal end **218** of the housing **211** would be integrally formed with the remainder of the body of the housing **211** in this variation of the puncturing device **200.** The skin puncturing assembly **215** may further include a protective tip guard **282** connected to a carrier member **250.** The tip guard **282** may be formed integrally with the body of the carrier member **250;** and may include a notched connection with the carrier member **250** in a similar manner as with protective cover **120** described above.

Flexure members **238** are formed or provided on a proximal end **244** of the shield **213.** The flexure members **238** define structure for retaining the lancet structure in an initial armed position retracted within the housing, acting in a similar manner as the pivotal lever element and the retaining hub of the embodiments previously described in connection with FIGS. 1-19. For example, projections **276** on the flexure members **238** extend inward so as to engage or coact with the carrier member **250** of the skin puncturing assembly **215.** The projections **276** engage or extend into a circumferential recess **200** defined or formed in the carrier member **250.** The recess **210** defines a circumferential edge **212,** which is engaged by the projections **276** of the flexure members **238.** The engagement edges **277** of the projections **276** in the lancet device **210** illustrated in FIGS. 23A-23D are formed or defined by a radially inward-extending tab **214** on each of the projections **276.** Engagement edges **277** act in a similar manner as shoulder **94** described above with reference to FIGS. 1-18, with the load represented by the skin puncturing assembly **215** resting on the engagement edges **277.**

The projections **276** maintain the carrier member **250,** and thus the skin puncturing assembly **215,** in the retracted position until released of engagement with the carrier member **250** by axial displacement of the shield **213** into the housing **211.** In the pre-actuated state of the lancet device **200,** the biasing force of the drive spring **270** is restrained by the projections **276** and by engagement of the distal end **242** of the shield **213** with an interfering structure provided at the distal end **216** of the housing **211.** In particular, while the shield **213** is axially or longitudinally movable or displaceable into the housing **211,** the shield **213** is prevented from moving distally relative to the housing **211** by an edge **217** formed on the shield **213.** The edge **217** is formed or defined by a portion **219** of the shield **213** having an increased wall thickness. The edge **217** coacts or engages with an internal lip **220** formed at the distal end **216** of the housing **211** to restrain the force of the drive spring **270.** The engagement of the edge **217** with the lip **220** allows the flexure members **238** to maintain the carrier member **250** in the retracted position and restrain the force of the drive spring **270.** In particular, in the pre-actuated state of the lancet device **200,** the force of the drive spring **270** is transmitted via the projections **276** to the body of the shield **213,** which causes the edge **217** on the shield **213** to engage the lip **220** and restrain the force of the drive spring **270.**

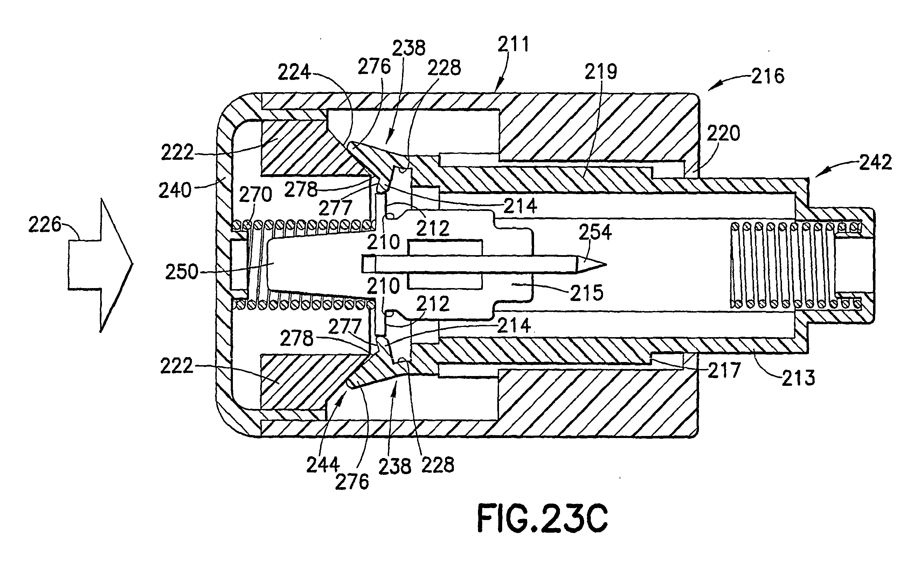

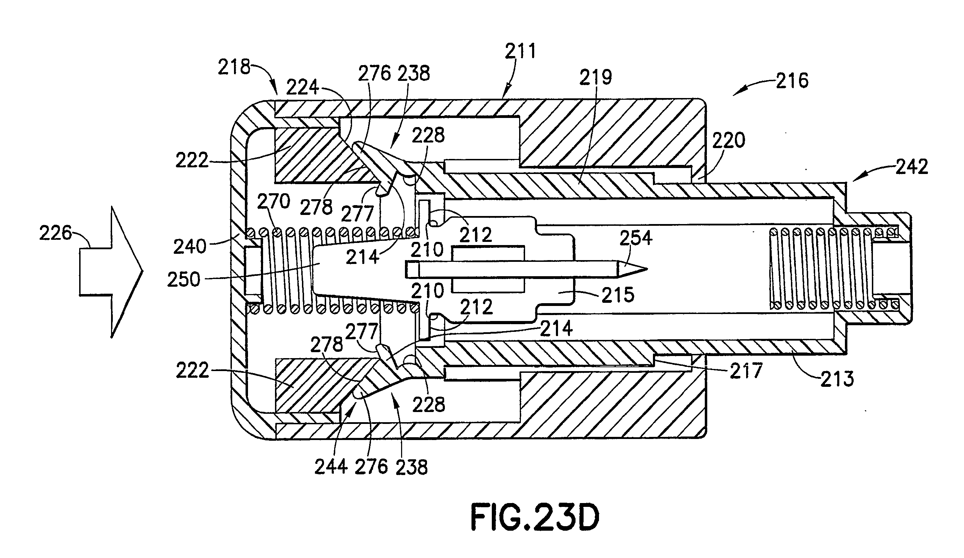

The flexure members **238** are adapted to be released of engagement with the carrier member **250** by one or more actuating members **222** (similar to the internal contact **46** in the embodiment described above with reference to FIGS. 1-18). The actuating member **222** may be formed integrally with the end cap **240,** or may be formed separately therefrom and secured to the end cap **240** with, for example, an adhesive. The actuating member **222** includes a tapered camming surface **224** (similar to engagement surface **47),** which is adapted to coact or engage with the tapered camming surfaces **278** of the flexure members **238** for actuating the puncturing device **200.** In particular, to actuate the puncturing device **200** shown in FIGS. 23A-23D, the user, typically a medical practitioner, places the distal end **242** of the shield **213** in contact with the body part where a blood sample is to be taken, and applies pressure in the direction of arrow **226** in FIGS. 23A-23D to cause the shield **213** to move proximally into the housing **211.** The movement of the shield **213** into the housing **211** causes the opposing camming surfaces **278, 224** on the flexure members **238** and actuating member **222,** respectively, to engage and interact. As the shield **213** is displaced or moved into the housing **211,** the flexure members **238** are flexed radially outward due to the interaction of the opposing camming surfaces **278, 224,** as shown in FIGS. 23C and 23D. The flexure members **238** may be adapted or configured to bend or break once they are flexed radially outward a preset distance, angle of rotation, or amount. For example, the flexure members **238** may be formed with a weakened area **228,** such as a score line, so that the flexure members **238** break when flexed radially outward a preset distance or degree of rotation. Moreover, weakened area **228** may act as a hinge in a similar manner as pivot hinge **98** described above.

During such movement of the flexure members **238,** projections **276** tilt toward the rear end cap **240,** thereby "lifting" or moving carrier member **250** toward rear cap **240** and compressing or further compressing drive spring **240.** Once the projections **276** on the flexure members **238** are released of engagement with the carrier member **250,** the drive spring **270** is free to move the carrier member **250** from the retracted position to the puncturing position. The drive spring **270** preferably has sufficient stored energy to cause the sharp distal tip **254** of the skin puncturing element **215** to pierce the skin of a person or animal once the flexure members **238** are released of engagement with the carrier member **250.**

As the carrier member **250** moves distally and reaches the puncturing position wherein the sharp distal tip **254** of the skin puncturing element **215** is fully exposed, a retraction spring **274** is compressed between the carrier member **250** and the distal end **242** of the shield **213** in a similar manner as retraction spring **110** discussed above with reference to FIGS. 1-18. The compression of the retraction spring **274** provides a return or retraction force that acts on the carrier member **250** to move the carrier member **250** in a return, proximal, or retraction direction in the housing **211,** which returns or retracts the skin puncturing element **215** and the sharp distal tip **254** thereof fully into the housing **211** and shield **213.** The retraction spring **274** thereafter prevents the reemergence of the skin puncturing element **215** from the housing **211** and shield **213.**

While specific embodiments of the lancet device have been described, those skilled in the art may make modifications and alterations without departing from the scope and spirit of the invention. Accordingly, the above detailed description is intended to be illustrative rather than restrictive. The invention is defined by the appended claims, and all changes to the invention that fall within the meaning and range of equivalency of the claims are to be embraced within their scope.

## Claims

1. A lancet device comprising:
a housing (12);
a lancet structure (70) comprising a puncturing element (72), the lancet structure disposed within the housing and adapted for movement between a pre-actuated position wherein the puncturing element is retained within the housing and a puncturing position wherein the puncturing element extends through a forward end (22) of the housing (12),
a drive spring (102) disposed between a rearward end of the housing and the lancet structure (70) for biasing the lancet structure toward the puncturing position; and
a lever element (92) pivotal about a fulcrum, the lever element providing interference engagement with the lancet structure (70) and retaining the lancet structure in the pre-actuated position and adapted to retain the lancet structure against the bias of the drive spring (102);
**characterized in that**
the lever element (92) comprises a class 1 type lever,
wherein movement of the housing (12) toward the lever element (92) causes a portion (94) of the lever element to pivot about the fulcrum (98), thereby moving the lancet structure (70) toward the rearward end (26) of the housing to at least partially compress the drive spring and releasing the interference engagement between the lever element and the lancet structure, permitting the drive spring (102) to drive the lancet structure through the housing toward the puncturing position.

2. A lancet device as in claim 1, further comprising an internal contact within the housing, wherein movement of the housing (12) toward the lever element (92) causes the internal contact within the housing to pivot a portion of the lever element about the fulcrum (98).

3. A lancet device as in claim 2, wherein the lever element comprises a shoulder (94) in interference engagement with the lancet structure (70) and a contact surface (96) for engagement with the internal contact (46) of the housing.

4. The lancet device of claim 3, wherein the internal contact (46) of the housing comprises an integrally formed cam surface (47) for cooperating engagement with the contact surface (96) of the lever element (92).

5. The lancet device of claim 3, wherein the lever element (92) comprises a wedge pivotally hinged to a retaining hub (90) forming a pivot hinge defining the fulcrum (98) for cooperative pivoting of the shoulder (94) and the contact surface (96).

6. The lancet device of claim 5, wherein the retaining hub (90) comprises an annular rim (100) with the lever (92) pivotally hinged to the annular rim.

7. The lancet device of claim 6, wherein the retaining hub (90) comprises a pair of levers (92) supported on opposing sides of the annular rim.

8. The lancet device of claim 3, wherein the lancet structure (70) further comprises a carrier element including a finger (82) in interference engagement with the shoulder (94) of the lever element.

9. The lancet device of claim 1, further comprising a shield (14) extending through the forward end (22) of the housing (12) and movable with respect to the housing, with the lever element (92) adjacent a rearward end of the shield.

10. The lancet device of claim 9, wherein the lever element (92) comprises a lever pivotally hinged to a retaining hub (90) with the lancet structure (70) retained by the lever element at the rearward end of the shield (14), and wherein movement of the rearward end of the shield toward the rearward end of the housing (12) causes the internal contact within the housing to pivot the lever of the retaining hub (90).

11. The lancet device of claim 10, wherein the lancet structure (70) and the shield (14) including corresponding guiding surfaces (78, 80) for guiding the lancet structure through the shield.

12. The lancet device of claim 9, wherein the shield (14) and the housing (12) including corresponding guiding surfaces (44, 46) for guiding the shield through the housing.

13. The lancet device of claim 9, further comprising a retraction spring (110) for retracting the lancet structure within the shield after the drive spring (102) drives the lancet structure through the shield (14) toward the puncturing position.

14. The lancet device of claim 9, further comprising locking structure extending between the shield (14) and the housing (12) for maintaining the shield in fixed relation to the housing after the drive spring (102) drives the lancet structure through the shield (14) toward the puncturing position.

15. The lancet device of claim 1, further comprising a lancet cover (120) removably covering the puncturing element (72) of the lancet structure (70).

16. The lancet device of one of claims 1-15, further comprising
a retaining hub (90) retaining the lancet structure (70) in the pre-actuated position against the bias of the drive spring (102), the retaining hub comprising said lever (92) pivotal about a fulcrum (98), the lever providing interference engagement with the lancet structure (70); and
an actuator (39, 46) adapted to pivot the lever (92) about the fulcrum, thereby releasing the lever from interference engagement with the lancet structure (70), permitting the drive spring (102) to drive the lancet structure toward the puncturing position.

17. A lancet device as in claim 16, wherein the actuator comprises an actuation element (25a) extending through and into the housing (12a), wherein movement of the actuation element with respect to the housing causes the actuation element to pivot the lever (92) about the fulcrum (98).

18. A lancet device as in claim 17, wherein the actuation element (25a) extends through a rearward end of the housing (12a) and is movable with respect to the housing to cause the actuation element to pivot the lever about the fulcrum.

19. A lancet device as in claim 16, wherein the actuator comprises an internal contact (46) integrally formed within the housing, wherein movement of the housing (12) toward the retaining hub (90) causes the internal contact within the housing to pivot the lever (92) about the fulcrum (98).

20. The lancet device of claim 19, further comprising a shield (14) extending through the forward end of the housing (12) and movable with respect to the housing, with the retaining hub (90) adjacent a rearward end of the shield.

21. The lancet device of claim 20, wherein the lancet structure (70) is retained by the retaining hub (90) at the rearward end of the shield (14), and wherein movement of the rearward end of the shield toward the rearward end of the housing (12) causes the actuator (39, 46) to pivot the lever (92) of the retaining hub.

22. The lancet device of claim 20, wherein the lancet structure (70) and the shield (14) including corresponding guiding surfaces (78, 80) for guiding the lancet structure through the shield.

23. The lancet device of claim 20, wherein the shield (14) and the housing (12) including corresponding guiding surfaces (44, 46) for guiding the shield through the housing.

24. The lancet device of claim 20, further comprising a retraction spring (110) for retracting the lancet structure within the shield after the drive spring (102) drives the lancet structure through the shield (14) toward the puncturing position.

25. The lancet device of claim 20, further comprising locking structure extending between the shield (14) and the housing (12) for maintaining the shield in fixed relation to the housing after the drive spring (102) drives the lancet structure through the shield (14) toward the puncturing position.

26. A lancet device as in claim 25, wherein the lever comprises a shoulder (94) in interference engagement with the lancet structure (70) and a contact surface (96) for engagement with the internal contact (46) of the housing.

27. The lancet device of claim 26, wherein the lever (92) comprises a wedge pivotally hinged to the retaining hub (90) forming a pivot hinge defining the fulcrum (98) for cooperative pivoting of the shoulder (94) and the contact surface (96).

28. The lancet device of claim 16, wherein the retaining hub comprises an annular rim (100) with the lever (92) pivotally hinged to the annular rim.

29. The lancet device of claim 28, wherein the retaining hub (90) comprises a pair of levers (92) supported on opposing sides of the annular rim (100).

30. The lancet device of claim 26, wherein the lancet structure further comprises a carrier element (76) including a finger (82) in interference engagement with the shoulder (94) of the lever (98).

## Patentansprüche

1. Lanzettenvorrichtung mit:
einem Gehäuse (12);
einer Lanzettenstruktur (70) mit einem Punktionselement (72), wobei die Lanzettenstruktur in dem Gehäuse angeordnet ist und zwischen einer vorbetätigten Position, in welcher das Punktionselement in dem Gehäuse zurückgehalten ist, und einer Punktionsposition bewegbar ist, in welcher sich das Punktionselement durch ein vorderes Ende (22) des Gehäuses (12) hindurch erstreckt,
einer zwischen einem hinteren Ende des Gehäuses und der Lanzettenstruktur (70) angeordneten Triebfeder (102) zum Vorspannen der Lanzettenstruktur in Richtung der Punktionsposition, und
einem um einen Schwenkpunkt schwenkbaren Hebelelement (92), wobei das Hebelelement in Passeingriff mit der Lanzettenstruktur (70) steht und die Lanzettenstruktur in der vorbetätigten Position zurückhält und zum Zurückhalten der Lanzettenstruktur entgegen der Vorspannung der Triebfeder (102) ausgebildet ist,
**dadurch gekennzeichnet, dass**
das Hebelelement (92) einen Hebel vom Klasse-1-Typ aufweist,
wobei die Bewegung des Gehäuses (12) in Richtung des Hebelements (92) das Schwenken eines Bereichs (94) des Hebelelements um den Schwenkpunkt (98) bewirkt, wodurch die Lanzettenstruktur (70) in Richtung des hinteren Endes (26) des Gehäuses bewegt wird, um die Triebfeder zumindest teilweise zusammenzudrücken, und der Passeingriff zwischen dem Hebelelement und der Lanzettenstruktur gelöst wird, wodurch es der Triebfeder (102) möglich ist, die Lanzettenstruktur durch das Gehäuse in Richtung der Punktionsposition zu treiben.

2. Lanzettenvorrichtung nach Anspruch 1, ferner mit einem inneren Kontakt in dem Gehäuse, wobei die Bewegung des Gehäuses (12) in Richtung des Hebelelements (92) bewirkt, dass der innere Kontakt im Gehäuse einen Bereich des Hebelelements um den Schwenkpunkt (98) schwenkt.

3. Lanzettenvorrichtung nach Anspruch 2, bei der das Hebelelement eine Schulter (94) in Passeingriff mit der Lanzettenstruktur (70) und eine Kontaktfläche (96) zum Angreifen an dem inneren Kontakt (46) des Gehäuses aufweist.

4. Lanzettenvorrichtung nach Anspruch 3, bei welcher der innere Kontakt (46) des Gehäuses eine einstückig ausgebildete Nockenfläche (47) zum zusammenwirkenden Angreifen an der Kontaktfläche (96) des Hebelelements (92) aufweist.

5. Lanzettenvorrichtung nach Anspruch 3, bei welcher das Hebelelement (92) einen Keil aufweist, der schwenkbar an einem Rückhaltekörper (90) angelenkt ist, welcher ein Schwenkgelenk bildet, das den Schwenkpunkt (98) zum zusammenwirkenden Verschwenken der Schulter (94) und der Kontaktfläche (96) bildet.

6. Lanzettenvorrichtung nach Anspruch 5, bei welcher der Rückhaltekörper (90) einen ringförmigen Kranz (100) aufweist, wobei der Hebel (92) schwenkbar an dem ringförmigen Kranz angelenkt ist.

7. Lanzettenvorrichtung nach Anspruch 6, bei welcher der Rückhaltekörper (90) zwei Hebel (92) aufweist, welche an gegenüberliegenden Seiten des ringförmigen Kranzes abgestützt sind.

8. Lanzettenvorrichtung nach Anspruch 3, bei welcher die Lanzettenstruktur (70) ferner ein Trägerelement mit einem Finger (82) aufweist, welcher in Passeingriff mit der Schulter (94) des Hebelelements steht.

9. Lanzettenvorrichtung mach Anspruch 1, ferner mit einem Schutz (14), der sich durch das vordere Ende (22) des Gehäuses (12) hindurch erstreckt und in bezug auf das Gehäuse bewegbar ist, wobei das Hebelelement (92) einem hinteren Ende des Schutzes benachbart ist.

10. Lanzettenvorrichtung nach Anspruch 9, bei welcher das Hebelelement (92) einen schwenkbar an einem Rückhaltekörper (90) angelenkten Hebel aufweist, wobei die Lanzettenstruktur (70) von dem Hebelelement am hinteren Ende des Schutzes (14) gehalten ist, und wobei die Bewegung des hinteren Endes des Schutzes in Richtung des hinteren Endes des Gehäuses (12) das Verschwenken des Hebels des Rückhaltekörperes (90) durch den inneren Kontakt in dem Gehäuse bewirkt.

11. Lanzettenvorrichtung nach Anspruch 10, bei welcher die Lanzettenstruktur (70) und der Schutz (14) entsprechende Führungsflächen (78, 80) zum Führen der Lanzettenstruktur durch den Schutz aufweisen.

12. Lanzettenvorrichtung nach Anspruch 9, bei welcher der Schutz (14) und das Gehäuse (12) entsprechende Führungsflächen (44, 46) zum Führen des Schutzes durch das Gehäuse aufweisen.

13. Lanzettenvorrichtung nach Anspruch 9, ferner mit einer Rückzugsfeder (110) zum Zurückziehen der Lanzettenstruktur in den Schutz, nachdem die Triebfeder (102) die Lanzettenstruktur durch den Schutz (14) in Richtung der Punktionsposition getrieben hat.

14. Lanzettenvorrichtung nach Anspruch 9, ferner mit einer Verriegelungsstruktur, die sich zwischen dem Schutz (14) und dem Gehäuse (12) erstreckt, um den Schutz in einem fixierten Verhältnis in bezug zu dem Gehäuse zu halten, nachdem die Triebfeder (102) die Lanzettenstruktur durch den Schutz (14) in Richtung der Punktionsposition getrieben hat.

15. Lanzettenvorrichtung nach Anspruch 1, ferner mit einer Lanzettenabdeckung (120), welche das Punktionselement (72) der Lanzettenstruktur (70) lösbar abdeckt.

16. Lanzettenvorrichtung nach einem der Ansprüche 1-15, ferner mit einem Rückhaltekörper (90), welcher die Lanzettenstruktur (70) entgegen der Vorspannung der Triebfeder (102) in der vorbetätigten Position hält, wobei der Rückhaltekörper den um einen Schwenkpunkt schwenkbaren Hebel (92) aufweist, wobei der Hebel in Passeingriff mit der Lanzettenstruktur (70) steht; und
einer Betätigungseinrichtung (39, 46), welche zum Schwenken des Hebels (92) um den Schwenkpunkt ausgebildet ist, wodurch der Hebel aus dem Passeingriff mit der Lanzettenstruktur (70) gelöst wird, so dass es der Triebfeder (102) möglich ist, die Lanzettenstruktur in Richtung der Punktionsposition zu treiben.

17. Lanzettenvorrichtung nach Anspruch 16, bei welcher die Betätigungseinrichtung ein Betätigungselement (25a) aufweist, das sich durch und in das Gehäuse (12a) erstreckt, wobei die Bewegung des Betätigungselements in bezug auf das Gehäuse bewirkt, dass das Betätigungselement den Hebel (92) um den Schwenkpunkt (98) schwenkt.

18. Lanzettenvorrichtung nach Anspruch 17, bei welcher das Betätigungselement (25a) sich durch ein hinteres Ende des Gehäuses (12a) erstreckt und in bezug auf das Gehäuse bewegbar ist, um das Betätigungselement zum Schwenken des Hebels um den Schwenkpunkt zu veranlassen.

19. Lanzettenvorrichtung nach Anspruch 16, bei welcher die Betätigungseinrichtung einen einstückig in dem Gehäuse ausgebildeten inneren Kontakt (46) aufweist, wobei die Bewegung des Gehäuses (12) in Richtung des Rückhaltekörperes (90) den inneren Kontakt im Gehäuse veranlasst, den Hebel (92) um den Schwenkpunkt (98) zu schwenken.

20. Lanzettenvorrichtung nach Anspruch 19, ferner mit einem Schutz (14), der sich durch das vordere Ende des Gehäuses (12) erstreckt und in bezug auf das Gehäuse bewegbar ist, wobei der Rückhaltekörper (90) einem hinteren Ende des Schutzes benachbart ist.

21. Lanzettenvorrichtung nach Anspruch 20, bei welcher die Lanzettenstruktur (70) durch den Rückhaltekörper (90) an dem hinteren Ende des Schutzes (14) gehalten ist, und wobei die Bewegung des hinteren Endes des Schutzes in Richtung des hinteren Endes des Gehäuses (12) die Betätigungseinrichtung (39, 46) veranlasst, den Hebel (92) des Rückhaltekörperes zu verschwenken.

22. Lanzettenvorrichtung nach Anspruch 20, bei welcher die Lanzettenstruktur (70) und der Schutz (14) entsprechende Führungsflächen (78, 80) zum Führen der Lanzettestruktur durch den Schutz aufweisen.

23. Lanzettenvorrichtung nach Anspruch 20, bei welcher der Schutz (14) und das Gehäuse (12) entsprechende Führungsflächen (44, 46) zum Führen des Schutzes durch das Gehäuse aufweisen.

24. Lanzettenvorrichtung nach Anspruch 20, ferner mit einer Rückzugsfeder (110) zum Zurückziehen der Lanzettenstruktur in den Schutz, nachdem die Triebfeder (102) die Lanzettenstruktur durch den Schutz (14) in Richtung der Punktionsposition getrieben hat.

25. Lanzettenvorrichtung nach Anspruch 20, ferner mit einer Verriegelungsstruktur, die sich zwischen dem Schutz (14) und dem Gehäuse (12) erstreckt, um den Schutz in einem fixierten Verhältnis in bezug zu dem Gehäuse zu halten, nachdem die Triebfeder (102) die Lanzettenstruktur durch den Schutz (14) in Richtung der Punktionsposition getrieben hat.

26. Lanzettenvorrichtung nach Anspruch 25, bei welcher der Hebel eine Schulter (94) in Passeingriff mit der Lanzettenstruktur (70) und eine Kontaktfläche (96) zum Angreifen an dem inneren Kontakt (46) des Gehäuses aufweist.

27. Lanzettenvorrichtung nach Anspruch 26, bei welcher der Hebel (92) einen Keil aufweist, der schwenkbar an einem Rückhaltekörper (90) angelenkt ist, welcher ein Schwenkgelenk bildet, das den Schwenkpunkt (98) zum zusammenwirkenden Verschwenken der Schulter (94) und der Kontaktfläche (96) bildet.

28. Lanzettenvorrichtung nach Anspruch 16, bei welcher der Rückhaltekörper einen ringförmigen Kranz (100) aufweist, wobei der Hebel (92) schwenkbar an dem ringförmigen Kranz angelenkt ist.

29. Lanzettenvorrichtung nach Anspruch 28, bei welcher der Rückhaltekörper (90) zwei Hebel (92) aufweist, welche an gegenüberliegenden Seiten des ringförmigen Kranzes (100) abgestützt sind.

30. Lanzettenvorrichtung nach Anspruch 26, bei welcher die Lanzettenstruktur ferner ein Trägerelement (76) mit einem Finger (82) aufweist, welcher in Passeingriff mit der Schulter (94) des Hebels (92) steht.

## Revendications

1. Dispositif lancette comprenant:
un boîtier (12);
une structure lancette (70) comprenant un élément de ponction (72), ladite structure lancette étant disposée dans ledit boîtier et étant apte à être déplacée entre une position pré-actionnée, dans laquelle l'élément de ponction est retenu dans ledit boîtier, et une position de ponction dans laquelle l'élément de ponction s'étend à travers une extrémité avant (22) dudit boîtier (12);
un ressort-moteur (102) disposé entre une extrémité arrière dudit boîtier et la structure lancette (70) pour précontraindre ladite structure lancette vers ladite position de ponction; et
un élément levier (92) pivotable autour d'un pivot, ledit élément levier étant ajusté en serrage avec ladite structure lancette (70) et retenant ladite structure lancette dans la position pré-actionnée et étant apte à retenir ladite structure lancette contre la précontrainte du ressort-moteur (102);
**caractérisé en ce que**
l'élément levier (92) comprend un levier du type classe 1,
le déplacement dudit boîtier (12) vers ledit élément levier (92) faisant une partie (94) de l'élément levier de pivoter autour du pivot (98), ainsi déplaçant la structure lancette (70) vers l'extrémité arrière (26) dudit boîtier afin de comprimer le ressort-moteur au moins partiellement et de relâcher l'engagement en serrage entre ledit élément levier et la structure lancette, permettant au ressort-moteur (102) d'entraîner la structure lancette à travers le boîtier vers la position de ponction.

2. Dispositif lancette selon la revendication 1, en outre comprenant un contact interne dans le boîtier, le déplacement dudit boîtier (12) vers l'élément levier (92) cause le contact interne dans le boîtier de pivoter une partie de l'élément levier autour le pivot (98).

3. Dispositif lancette selon la revendication 2, dans lequel l'élément levier comprend un épaulement (94) engagé en serrage avec la structure lancette (70) et une face de contact (96) pour s'engager au contact interne (46) dudit boîtier.

4. Dispositif lancette selon la revendication 3, dans lequel ledit contact interne (46) dudit boîtier comprend une face de came (47) d'un seul tenant avec ledit boîtier, pour l'engagement coopérant avec la face de contact (96) dudit élément levier (92).

5. Dispositif lancette selon la revendication 3, dans lequel ledit élément levier (92) comprend une cale articulée de manière pivotable à une bague de retenue (90) formant un joint de pivotement qui définit le pivot (98) pour le pivotement coopératif de l'épaulement (94) et de la face de contact (96).

6. Dispositif lancette selon la revendication 5, dans lequel la bague de retenue (90) comprend une couronne annulaire (100), le levier (92) étant articulé de manière pivotable à ladite couronne annulaire.

7. Dispositif lancette selon la revendication 6, dans lequel la bague de retenue (90) comprend deux leviers (92) supportés à des côtés opposés de la bague de retenue.

8. Dispositif lancette selon la revendication 3, dans lequel la structure lancette (70) en outre comprend un élément porteur avec un doigt (82) engagé en serrage avec l'épaulement (94) de l'élément levier.

9. Dispositif lancette selon la revendication 1, en outre comprenant un protecteur (14) s'étendant à travers l'extrémité avant (22) dudit boîtier (12) et étant déplaçable par rapport audit boîtier, ledit élément levier (92) étant situé adjacent une extrémité arrière du protecteur.

10. Dispositif lancette selon la revendication 9, dans lequel ledit élément levier (92) comprend un levier articulé de manière pivotable à une bague de retenue (90), la structure lancette (70) étant retenue par l'élément levier à ladite extrémité arrière du protecteur (14), et le déplacement de ladite extrémité arrière du protecteur vers ladite extrémité arrière du boîtier (12) causant le contacte interne dans le boîtier à pivoter le levier de la bague de retenue (90).

11. Dispositif lancette selon la revendication 10, dans lequel la structure lancette (70) et le protecteur (14) comprennent des faces de guidage (78, 80) correspondantes pour guider la structure lancette à travers le protecteur.

12. Dispositif lancette selon la revendication 9, dans lequel le protecteur (14) et ledit boîtier (12) comprennent des faces de guidage (44, 46) correspondantes pour guider le protecteur à travers le boîtier.

13. Dispositif lancette selon la revendication 9, en outre comprenant un ressort de retour (110) pour retourner ladite structure lancette dans le protecteur après le ressort-moteur (102) a déplacé ladite structure lancette à travers ledit protecteur (14) vers la position de ponction.

14. Dispositif lancette selon la revendication 9, en outre comprenant une structure de verrouillage s'étendant entre le protecteur (14) et le boîtier (12) pour maintenir le protecteur dans un rapport fixe relatif au boîtier après le ressort-moteur (102) a déplacé ladite structure lancette à travers le protecteur (14) vers la position de ponction.

15. Dispositif lancette selon la revendication 1, en outre comprenant une chape de lancette (120) couvrant l'élément de ponction (72) de la structure lancette (70) de manière amovible.

16. Dispositif lancette selon l'une des revendications 1-15, en outre comprenant une bague de retenue (90) retenant la structure lancette (70) dans la position préactionnée contre la précontrainte du ressort-moteur (102), ladite bague de retenue comprenant ledit levier (92) pivotable autour d'un pivot (98), le levier étant engagé en serrage avec la structure lancette (70); et
un actionneur (39, 46) apte à pivoter le levier (92) autour dudit pivot, ainsi relâchant le levier de son engagement en serrage avec ladite structure lancette (70), permettant au ressort-moteur (102) de déplacer la structure lancette vers la position de ponction.

17. Dispositif lancette selon la revendication 16, dans lequel l'actionneur comprend un élément d'actionnement (25a) s'étendant à travers et dans le boîtier (12a), le déplacement dudit élément d'actionnement par rapport au boîtier causant l'élément d'actionnement de pivoter le levier (92) autour du pivot (98).

18. Dispositif lancette selon la revendication 17, dans lequel l'élément d'actionnement (25a) s'étend à travers une extrémité arrière du boîtier (12a) et est déplaçable par rapport au boîtier afin de causer l'élément d'actionnement de pivoter le levier autour du pivot.

19. Dispositif lancette selon la revendication 16, dans lequel l'actionneur comprend un contact interne (46) formé d'un seul tenant avec le boîtier, le déplacement du boîtier (12) vers la bague de retenue (90) causant le contact interne dans le boîtier de pivoter le levier (92) autour du pivot (98).

20. Dispositif lancette selon la revendication 19, en outre comprenant un protecteur (14) s'étendant à travers l'extrémité avant dudit boîtier (12) et déplaçable par rapport au boîtier, la bague de retenue (90) étant située adjacente une extrémité arrière du protecteur.

21. Dispositif lancette selon la revendication 20, dans lequel la structure lancette (70) est retenue par la bague de retenue (90) à l'extrémité arrière du protecteur (14), et le déplacement de l'extrémité du protecteur vers l'extrémité arrière du boîtier (12) causant l'actionneur (39, 46) de pivoter le levier (92) autour de la bague de retenue.

22. Dispositif lancette selon la revendication 20, dans lequel la structure lancette (70) et le protecteur (14) comprennent des faces de guidage (78, 80) correspondantes pour guider la structure lancette à travers le protecteur.

23. Dispositif lancette selon la revendication 20, dans lequel le protecteur (14) et ledit boîtier (12) comprennent des faces de guidage (44, 46) correspondantes pour guider le protecteur à travers le boîtier.

24. Dispositif lancette selon la revendication 20, en outre comprenant un ressort de retour (110) pour retourner ladite structure lancette dans le protecteur après le ressort-moteur (102) a déplacé ladite structure lancette à travers ledit protecteur (14) vers la position de ponction.

25. Dispositif lancette selon la revendication 20, en outre comprenant une structure de verrouillage s'étendant entre le protecteur (14) et le boîtier (12) pour maintenir le protecteur dans un rapport fixe relatif au boîtier après le ressort-moteur (102) a déplacé ladite structure lancette à travers le protecteur (14) vers la position de ponction.

26. Dispositif lancette selon la revendication 25, dans lequel le levier comprend un épaulement (94) engagé en serrage avec la structure lancette (70) et une face de contact (96) pour s'engager au contact interne (46) dudit boîtier.

27. Dispositif lancette selon la revendication 26, dans lequel ledit levier (92) comprend une cale articulée de manière pivotable à une bague de retenue (90) formant un joint de pivotement qui définit le pivot (98) pour le pivotement coopératif de l'épaulement (94) et de la face de contact (96).

28. Dispositif lancette selon la revendication 16, dans lequel la bague de retenue comprend une couronne annulaire (100), le levier (92) étant articulé de manière pivotable à ladite couronne annulaire.

29. Dispositif lancette selon la revendication 28, dans lequel la bague de retenue (90) comprend deux leviers (92) supportés à des côtés opposés de la bague de retenue (100).

30. Dispositif lancette selon la revendication 26, dans lequel la structure lancette en outre comprend un élément porteur (76) avec un doigt (82) engagé en serrage avec l'épaulement (94) de l'élément levier (98).
